# EUROPÄISCHE PATENTSCHRIFT

(11) **EP 3 490 513 B1**
(45) Veröffentlichungstag und Bekanntmachung des Hinweises auf die Patenterteilung: **05.10.2022**
(21) Anmeldenummer: 17749413.5
(22) Anmeldetag: 26.07.2017
(51) Int. Cl.: A61F 9/008

(54) **OPHTHALMOLOGISCHE LASERTHERAPIE MIT NACHFÜHRSYSTEM**
OPHTHALMOLOGICAL LASER THERAPY WITH TRACKING SYSTEM
DISPOSITIF DE THÉRAPIE PAR LASER OPHTALMOLOGIQUE POURVU DE SYSTÈME SUIVEUR

(30) Priorität: 29.07.2016 DE 102016214087
(43) Veröffentlichungstag der Anmeldung: 05.06.2019
(73) Patentinhaber: Carl Zeiss Meditec AG, 07745 Jena (DE)
(72) Erfinder: RILL, Michael Stefan, 07751 Jena (DE); BERGT, Michael, 99425 Weimar (DE)
(74) Vertreter: Rößner, Ulrike
(86) Internationale Anmeldenummer: PCT/EP2017/068892
(87) Internationale Veröffentlichungsnummer: WO 2018/019890

(56) Entgegenhaltungen:
- WO-A2-2016/058931
- US-A1- 2014 200 563
- US-A1- 2014 276 674
- US-A1- 2015 245 944

## Beschreibung

Die vorliegende Erfindung betrifft ein Nachführsystem für eine ophthalmologische Lasertherapievorrichtung zur Nachregelung einer Therapieführung zur Erzeugung einer Struktur mittels einer Laserstrahlung der ophthalmologischen Lasertherapievorrichtung in einem Gewebe eines Auges, eine ophthalmologische Lasertherapievorrichtung mit einer Einrichtung zur Erzeugung einer Laserstrahlung, einer Optik zur Fokussierung der Laserstrahlung in ein Bearbeitungsvolumen, einer Einrichtung zur Veränderung der Lage des Fokus und einer Steuereinrichtung.

Ophthalmologische Lasertherapievorrichtungen werden heute an vielen Stellen zur Korrektur von Fehlsichtigkeiten aber auch zur Therapie von anderen Augenerkrankungen eingesetzt. Dabei werden beispielsweise Teile des Augengewebes lokal verändert, die anschließend extrahiert werden können, oder aber Schnitte gesetzt, die eine Fehlsichtigkeit durch eine Entspannungswirkung der Cornea korrigieren. Ablösungserscheinungen können ebenfalls durch Laser mit Koagulationswirkung korrigiert werden. Sehr häufig werden ophthalmologische Lasertherapievorrichtungen für Katarakt-Operationen eingesetzt, bei denen die getrübte Linse eines Patientenauges entfernt und eine Intra-Okular-Linse (IOL) an deren Stelle eingesetzt wird.

Durch Photodisruption mit einer Laserstrahlung erzeugte Schnittfiguren sind dabei die häufigsten Anwendungen einer ophthalmologischen Lasertherapievorrichtung. Hierfür wird eine in das betreffende Augengewebe hinein fokussierte, gepulste Laserstrahlung verwendet, die in der Regel durch einen Femtosekunden-Laser oder einen Pikosekunden-Laser erzeugt wird. Durch eine Laserstrahlung können jedoch auch Augenstrukturen ablatiert oder aber strukturierte Gewebeveränderungen bzw. Gewebeverklebungen durch eine Koagulationswirkung erzeugt werden.

Während des Fortschritts des Einbringens einer Struktur mit einer Laserstrahlung in ein Augengewebe, beispielsweise einer Schnittfigur in die Cornea, die Sklera oder in die Linse und/oder ihre Linsenkapsel, kann sich das Ziel aufgrund von Augenbewegungen von der initialen Position fortbewegen. Selbst bei einer Fixierung des Auges relativ zur ophthalmologischen Lasertherapievorrichtung, wobei eine solche Fixierung mit einem Kontaktglas oder einem Flüssigkeits-Patienteninterface realisiert werden kann, ist ein solches Driften beispielsweise aufgrund einer Atembewegung möglich. Die mit der Laserstrahlung der ophthalmologischen Lasertherapievorrichtung währenddessen erzeugte Struktur weicht dann von der geplanten Struktur ab. Die erzeugte Struktur resultiert dabei aus einer Überlagerung der Bewegung des Fokus der Laserstrahlung entlang eines für diese Struktur kodierten Scanmusters und der unbeabsichtigten Bewegung des zu therapierenden Patientenauges.

Wenn nun die Erzeugung dieser Struktur, insbesondere einer Schnittfigur bzw. einer Schnittlinie, an einem Startpunkt beginnt, und der Endpunkt der Erzeugung der Struktur mit der fokussierten Laserstrahlung mit diesem Startpunkt zusammenfallen soll, so dass im Ergebnis eine geschlossene Struktur, ein eingeschlossenes Volumen oder eine eingeschlossene Fläche separiert wird, führt eine Bewegung des zu therapierenden Patientenauges dazu, dass sich der Startpunkt und der Endpunkt der Struktur nicht mehr treffen. Treffen sich Anfang und Ende einer ursprünglich Schnittfigur in einem Gewebe eines Patientenauges nicht mehr, so wird das Volumen innerhalb dieser Schnittfigur nicht mehr vollständig vom außerhalb der Schnittfigur befindlichen Gewebe getrennt. Dies kann ein Problem bei keratoplastischen Operationen in der Cornea sein, aber insbesondere bei der Ausführung einer sogenannten Kapsulotomie bei Katarakt-Operationen.

Es gibt verschiedene Varianten, ein Scanmuster für die Erzeugung einer Struktur zu erstellen. Sie sollen am Kapsulotomie-Schnitt erläutert werden:
Bei Multi-Path-Schnitten, insbesondere für die Kapsulotomie, wird die beabsichtigte laterale Schnittfigur, also die Schnittfigur wie sie in einer Aufsicht erkennbar ist, mehrmals hintereinander in leicht variierenden Tiefen im Zielobjekt wiederholt. Damit wird zum einen die Tiefenausdehnung des Zielobjekts überspannt und damit ein durchgehender Schnitt ermöglicht. Zudem werden toleranzbedingte Abweichungen der Zielobjekttiefe und der Schnitttiefe überbrückt. Da unterschiedliche Tiefen des Zielobjekts mit unterschiedlichen Teilen der hintereinander wiederholten Schnittfigur erzeugt werden, kommt es bei einer Bewegung des Zielobjekts zu unverbundenen Stücken der Schnittfläche bzw. zu Mehrfachausführungen des Schnittes, da dann die Schnittfigur nicht mehr exakt wiederholt sondern leicht - zumeist lateral - versetzt entsteht. Es entsteht also ggf. eine "unsaubere" Schnittfläche.

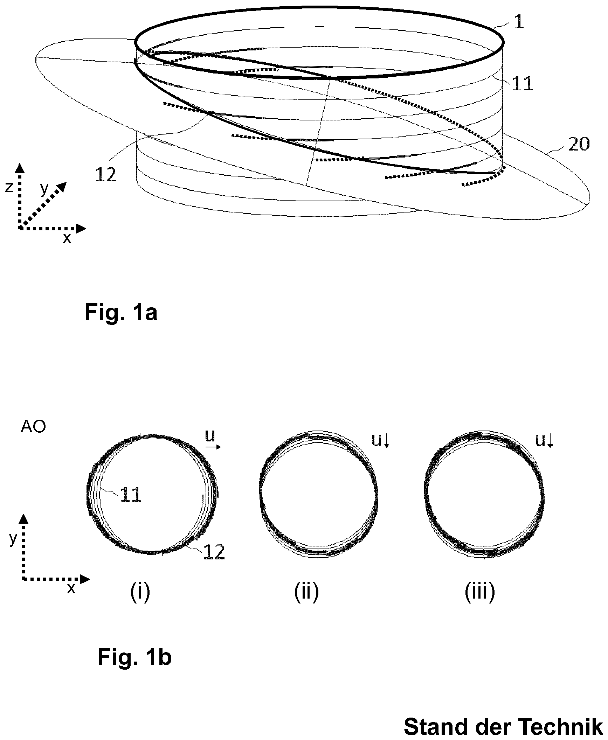

Die Fig. 1a stellt eine solche Situation bei Multipath-Schnitten nach dem Stand der Technik dar. Die Schnittfläche der Schnittfigur 1 durchdringt die zu schneidende Fläche 20 entlang einer räumlichen Bahn. Wird die Schnittfläche aus mehrmals wiederholten Schnittlinien 11 mit leichtem vertikalen Versatz bzw. helixartig aufgebaut, durchdringt jede Schnittlinie in einer vertikalen Höhe nur auf einem Teilsektor 12 nahe der räumlichen Durchdringungsbahn die zu schneidende Fläche 20 und bewirkt dort eine Schnittwirkung. Kommt es während des Schnittes zum Versatz der Schnittfläche, weisen die Teilsektoren 12 einen Versatz zueinander auf.

Die Fig. 1b zeigt verschiedene Möglichkeiten des Versatzes der Schnittlinien in einer Aufsicht AO auf die Schnittfläche der Schnittfigur 1 und der resultierenden Schnittlinien der zu schneidenden Fläche 20 während der Ausführung des Schnitts bei lateralem Versatz um den Vektor u wie in den jeweiligen Figuren (i), (ii), (iii) aufgezeigt. Zwischen den einzelnen Teilsektoren 12 kommt es sowohl zu Lücken als auch zum Überlapp je nach Richtung des Versatzes u. Die Versatz-Positionen bei der Erzeugung einer Struktur in einem Gewebe eines Patientenauges sind apriori nicht bekannt.

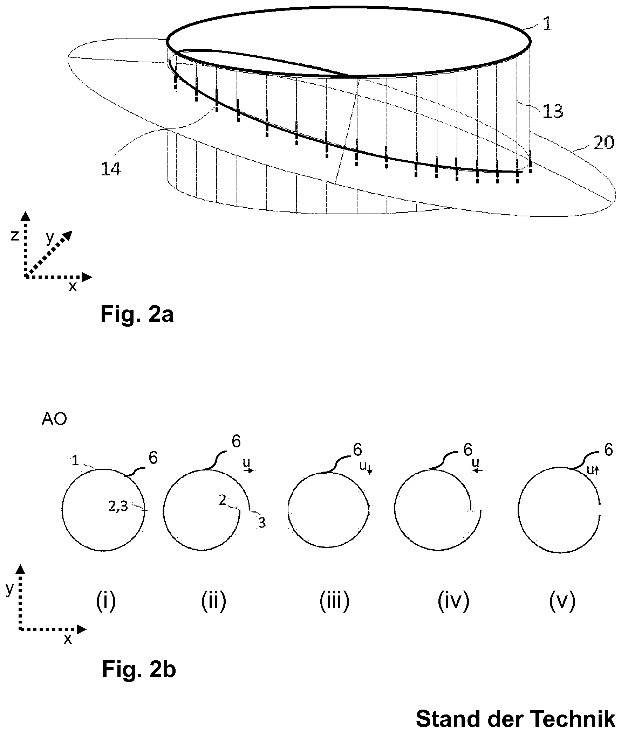

Bei Single-Path-Schnitten nach dem Stand der Technik wird, wie in Fig. 2a und 2b gezeigt, die beabsichtigte Schnittfigur 1 durch einmaliges langsames Überstreichen bzw. "Abfahren" einer in einer Aufsicht auf die Schnittfigur 1 sichtbaren Linie, einer Spur 6, bei schnellem Überstreichen einer zur Projektionsfläche der Aufsicht geneigten Fläche, zumeist einer zur Projektionsfläche senkrechten Fläche, entlang dieser Linie, ausgeführt. Im Stand der Technik entspricht die Projektionsfläche dabei der lateralen Ebene, d.h. einer zur optischen Achse senkrechten Ebene. Die Schnittfigur wird durch schnelles Überstreichen eines Tiefenbereiches während eines langsamen Überstreichens der in der Aufsicht sichtbaren Spur 6 ausgeführt. Wie in der Fig. 2a dargestellt, wird die Schnittfläche der Schnittfigur 1 aus benachbarten, in diesem Falle vertikalen, Geraden 13 entlang einer (Schnitt)-Linie aufgebaut. Die Schnittfigur 1 soll in diesem Fall in der Aufsicht kreisförmig und notwendigerweise geschlossen sein. Die Geraden 13 durchdringen die zu schneidende Fläche 20 nur in Teil-Höhenabschnitten 14 und bewirken dort eine Schnittwirkung.

Das Single-Path-Schnitt-Konzept hat aufgrund dieser Eigenschaften wie auch aufgrund der dadurch möglichen abweichenden Ausgestaltung einer ophthalmologischen Lasertherapievorrichtung, die ein solches Single-Path-Schnitt-Konzept erlaubt, Vorteile gegenüber dem Multi-Path-Schnitt-Konzept. Zu nennen ist hier vor allem die bessere Schnittqualität, also eine definierte und vollständige Trennung von Gewebe.

Benachbarte Geraden 13 werden bei Single-Path-Schnitten in zeitlich kurzem Abstand ausgeführt und weisen daher auch bei einem zeitlich veränderlichen Versatz der Schnittfläche 20 während des Schneidens relativ zur vorhergehenden Geraden 13 nur einen geringen Versatz auf. Erst wenn das Ende der Schnittlinie wieder den Anfang treffen muss, macht sich ein Gesamtversatz, also der Versatz der zwischen dem Beginn und dem Abschluss der Erzeugung der Schnittfigur 1 entstanden ist, bemerkbar.

Ohne weitere Maßnahmen würden sich bei Single-Path-Schnitten Anfang und Ende der Schnittfigur 1 bei einer Bewegung des Patientenauges, aber auch bei Bewegungen durch andere Einflüsse während des Einsatzes der ophthalmologischen Lasertherapievorrichtung nicht treffen. Der Schnitt wäre unvollständig.

Die Fig. 2b zeigt eine Aufsicht AO auf eine solche ursprünglich kreisförmige Schnittfigur 1, wie sie z.B. für die Kapsulotomie, also die Öffnung des anterioren Kapselsacks der Augenlinse, eingesetzt wird. Sie beginnt an einem Startpunkt 2 und endet am Endpunkt 3. Bewegt sich das geschnittene Objekt während der Schnittdauer nicht, ergibt sich eine geschlossene Schnittfigur 1: Startpunkt 2 und Endpunkt 3 der Spur 6 treffen sich, siehe Fig. 2b(i). Bewegt sich das Objekt jedoch während der Schnittdauer um den Vektor u, können sich je nach Bewegungsrichtung auch ungeschlossene Schnittfiguren, wie in Fig. 2b(ii)-(v) gezeigt, ergeben, bei denen dann der innere kreisförmige Teil nicht wie beabsichtigt entnommen werden kann.

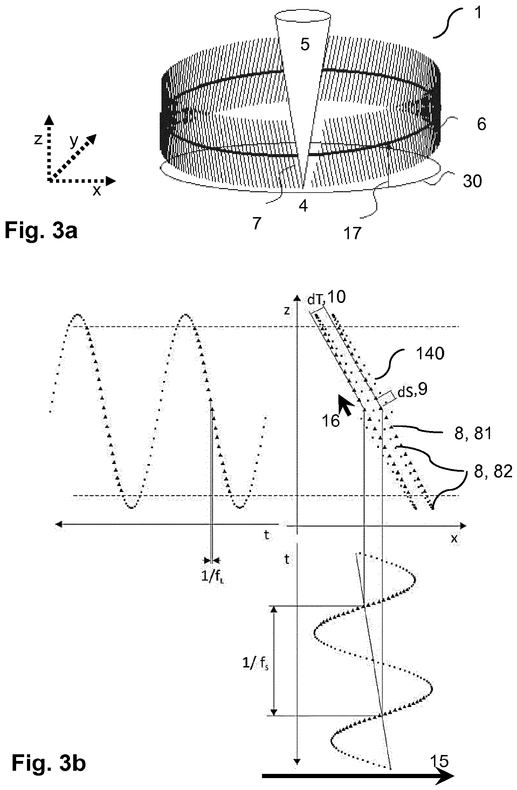

Vorteilhaft werden Single-Path-Schnitte mit einem Laserschnitt-Führungskonzept ausgeführt wie in der Fig. 3a und 3b und ausführlich in der DE 10 2015 212 877.1 beschrieben, die durch Bezugnahme Bestandteil dieser Beschreibung wird. Anstelle von vertikalen Schnittgeraden 13, wie in der Fig. 2a gezeigt, werden hier schräge Schnittlinien nebeneinandergesetzt, sogenannte Strokes 7. Die Fig. 3a zeigt die vorteilhafte Aneinanderreihung geneigter Strokes 7 zur Bearbeitung einer in sich geschlossenen, zylinderförmigen Fläche bzw. Struktur 1. Dabei werden die Strokes 7 so aneinandergereiht, dass bereits bestehende Strokes 7 aus dem bereits realisierten Teil des Scanmusters nicht den Fokuskegel 5 der Laserstrahlung durchdringen. Die Erzeugung der Struktur 1 wird somit nicht durch Abschattungen, die von bereits bestehenden Strokes 7 erzeugt werden, behindert.

Solch ein Scanmuster 140, in diesem Fall mit gleichgerichteten geneigten Strokes 7, wird beispielsweise durch synchrone Richtungswechselbewegungen 16, insbesondere durch synchrone oszillatorische Bewegungen eines schnellen z-Scanners und mindestens eines schnellen Lateralscanners, und gleichzeitiger langsamer Veränderung 15 des Schwingungsmittelpunktes erzeugt, wie in der Fig. 3b dargestellt. Die Bahn des Laserfokus 4 beschreibt dabei das Scanmuster 140.

Die hier dargestellten Punkte stellen zunächst potentielle Schusspositionen eines hierfür genutzten Kurzpulslasers aufgrund von dessen Repetitionsrate, also Laserfokuswirkbereiche 8, dar. Gibt der Laser Pulse mit einer festen Repetitionsrate ab, werden im Raum Laserfokuswirkbereiche 8 mit einem Spot-zu-Spot-Abstand, also einem Abstand zweier Laserfokuswirkbereiche 9, hinterlassen. Die Spot-zu-Spot-Abstände 9 variieren mit der Momentan-Position der Sinusschwingung und sind im Nulldurchgang der Schwingung am größten und in den Umkehrpunkten fast null.

Dabei markieren die Dreiecke die tatsächlich realisierten, also nicht geblockten, Laserfokuswirkbereiche 8, 81. In diesem Fall werden Laserfokuswirkbereiche nur in der schnellen Aufwärtsbewegung 16 realisiert. Laserpulse in den Umkehrpunkten der Sinus-Bewegung, die Laserfokuswirkbereiche 8, 82 in zu großer Nähe zueinander erzeugen würden, sowie in der Abwärtsbewegung werden in diesem Beispiel ausgeblendet. Der Abstand zweier geneigter Strokes 7 ist, da hier nur in einer Schwingungsrichtung geschnitten wird, über eine Schwingung konstant.

Trotzdem besteht hier gleichfalls das Problem des "Wegbewegens" der Schnittfigur aufgrund von Augenbewegungen, aber ggf. auch anderen aufgrund anderer Einflüsse.

In der WO 2016/058931 A2, wie auch in weniger schnittgeometrieerhaltender Art und Weise in der US2015/0245944 A1, wird vorgeschlagen, dieses Problem, das besonders nachteilig bei Nutzung des Single-Path-Schnitt-Konzepts auftritt, mittels einer Schnittüberlappung zu beheben. Dieser Lösungsansatz funktioniert prinzipiell. Es ist jedoch schwierig zu bestimmen, wie sehr sich die Schnittlinien überschneiden müssen, da sich die Bewegungsamplitude des Auges im Einzelfall nicht abschätzen lässt. Zudem führt dieses Verfahren zu einer Abweichung der Schnittform beispielsweise von der ideal für die Kapsulotomie gewünschten runden Schnittgeometrie, die bei einer solchen Kapsulotomie den Kapselsack ggf. destabilisieren könnte.

Die Kapsulotomie ist ein besonders wichtiger Schnitt der laserbasierten Katarakt-Operation, bei der der anteriore Teil des Kapselsacks kreisrund aufgeschnitten wird. Im nachfolgenden manuellen Teil der Operation, wird das Kapsulotomie-Häutchen herausgezogen und der Linsenkern des Auges durch die entstehende Öffnung hindurch beispielsweise mittels Ultraschall zerkleinert oder aber die bereits durch Photodisruption mittels der fokussierten Laserstrahlung vorfragmentierte Linse weiter zertrümmert und abgesaugt. Wird die Kapsulotomie so durchgeführt, dass die Schnittkurve offen bleibt, und das Kapsulotomie-Häutchen nicht richtig abgetrennt wird, wie in der Fig. 2b (ii), (iv) oder (v) gezeigt, können sich daraus fatale Komplikationen, wie z.B. nicht-kontrollierbare kapsuläre Risse, im manuellen Teil der Operation ergeben.

Zur Minimierung von potentiellen Risiken und für das optimale refraktive Ergebnis der Katarakt-Operation ist es zudem wichtig, dass die Kapsulotomie möglichst rund ist bzw. eine hohe Zirkularität aufweist. Einerseits wird dadurch das Einreißen des Kapselsacks während des manuellen Operationsteils unwahrscheinlicher. Eine Kapsulotomie mit einer hohen Zirkularität garantiert zudem eine sichere Abtrennung und Entfernung des Kapsulotomie-Häutchens. Auch trägt die hohe Zirkularität zur Vorhersehbarkeit der effektiven Position der implantierten Intraokularlinse bei.

Wie hier im Beispiel der Kapsulotomie ist auch bei der Erzeugung anderer Strukturen eine Zielformtreue der erzeugten Struktur im Hinblick auf die beabsichtigte Struktur von essentieller Bedeutung. Weitere Beispiele sind etwa die Separierung und anschließende Extraktion eines Lentikels in der Cornea oder eine Formgebung der Cornea mittels Ablation, aber auch, wenn die Augenlinse durch Photodisruption so vorgeschnitten werden soll, dass sie im Anschluss mit sehr geringer Ultraschallleistung zertrümmert und schließlich abgesaugt werden kann.

Aufgabe der vorliegenden Erfindung ist es deshalb, eine ophthalmologische Lasertherapievorrichtung bzw. eine Einrichtung für eine ophthalmologische Lasertherapievorrichtung zu beschreiben, die eine hohe Zielformtreue garantieren, selbst wenn sich die Position des Auges relativ zur ophthalmologischen Lasertherapievorrichtung, beispielsweise durch nichtvorhersehbare Augenbewegungen, während des Therapiefortschritts ändert.

Als eine mögliche Anwendung soll insbesondere die Vollständigkeit eines Kapsulotomieschnitts bei einer Katarakt-Operation gewährleistet werden.

Diese Aufgabe wird gelöst durch ein Nachführsystem für eine ophthalmologische Lasertherapievorrichtung zur Nachregelung einer Therapieführung nach Anspruch 1 und eine ophthalmologische Lasertherapievorrichtung nach Anspruch 10.

Ein Nachführsystem für eine ophthalmologische Lasertherapievorrichtung zur Nachregelung einer Therapieführung zur Erzeugung einer Struktur mittels einer Laserstrahlung der ophthalmologischen Lasertherapievorrichtung in einem Gewebe eines Patientenauges, bzw. allgemeinhin in einem Bearbeitungsvolumen der ophthalmologischen Lasertherapievorrichtung, umfasst eine Kontrolleinheit.

Üblicherweise wird bei einer ophthalmologischen Lasertherapie mit einer entsprechenden Vorrichtung nach dem Stand der Technik von einer festen Relativbeziehung zwischen dem Auge des Patienten und der ophthalmologischen Lasertherapievorrichtung, und hier insbesondere einer Nulllage eines Laserstrahls, also der Lage des nicht ausgelenkten Laserstrahls, ausgegangen. Vor Beginn der Therapie werden beide Systeme zueinander kalibriert, danach fährt der Therapielaserstrahl ein Scanmuster unter Zugrundelegung dieser Kalibrierung ab. Um diese feste Relativbeziehung zu erhalten, wird das Auge mit einem Patienteninterface, beispielsweise einem Flüssigkeitspatienteninterface oder einem Kontaktglas, an der ophthalmologischen Lasertherapievorrichtung fixiert. Wie oben schon beschrieben, kann es aber unter Umständen, insbesondere bei längeren Therapiezeiten, trotzdem zu Augenbewegungen oder aber anderweitigen Veränderungen der relativen Lage von Patientenauge und ophthalmologischen Lasertherapievorrichtung kommen.

Das Nachführsystem ermöglicht nun die aktive Nachregelung der Relativbeziehung zwischen dem Auge des Patienten und der ophthalmologischen Lasertherapievorrichtung, indem es diese unbeabsichtigten Änderungen feststellt und dafür sorgt, dass dem entgegengewirkt und damit die ursprüngliche relative Lage zwischen dem Patientenauge und der ophthalmologischen Lasertherapievorrichtung - direkt oder indirekt - wieder hergestellt wird. Ein Gewebe eines Auges kann dabei die Cornea, die Sklera, die Linse und ihr Kapselsack, aber auch der Glaskörper sein. Auch künstliche Materialien sind unter dem Begriff Gewebe eines Auges zu fassen. Beispielsweise kann auch ein entsprechendes Implantat nachgearbeitet werden.

Der Begriff "Struktur" umfasst hier verschiedene Ausgestaltungen: Eine wichtige Ausgestaltung ist die Erzeugung von Schnitten mittels Photodisruption oder mittels Ablation jeweils mit einer hierzu geeigneten Laserstrahlung: Während für die Photodisruption gepulste Laser genutzt werden, ist ein geeigneter Laser für die Ablation ein Excimerlaser (UV-Laser). Mittels Ablation kann ebenfalls strukturiert bzw. modelliert werden, und Gewebe gezielt auf einer größeren Fläche abgetragen werden. Für eine Gewebeveränderung, beispielsweise durch Koagulation, kommen hingegen YAG-Laser bzw. andere Laser im sichtbaren und/oder im Infrarotbereich zum Einsatz. Damit kann Gewebe strukturiert, verklebt oder aber anderweitig in seiner Grundstruktur verändert werden.

Erfindungsgemäß ist nun die Kontrolleinheit eingerichtet, Bilder und/oder Daten einer Untersuchungseinheit zur Charakterisierung einer Fläche oder eines Volumens des Auges zu empfangen und zu verarbeiten. Eine solche Untersuchungseinheit, die eine Fläche oder ein Volumen des Auges während der Lasertherapie beobachtet, kann dabei entweder Bilder oder ortsaufgelöste Daten oder aber beides gleichzeitig liefern. Diese Bilder und/oder Daten können Informationen zu Strukturen des Auges, Grenzflächen verschiedener Gewebearten des Auges oder aber Informationen zu künstlichen Markierungen beinhalten. Die Untersuchungseinheit sollte dabei das Auge eines Patienten über eine entsprechend große Fläche bzw. über ein großes Volumen charakterisieren, so dass sämtliche Relativbewegungen des Auges, wie beispielsweise ein Driften aufgrund von Atembewegungen oder aber anderen natürlichen Augenbewegungen, zu einem maximalen Versatz führen, der signifikant kleiner ist als der Durchmesser der beobachteten Fläche bzw. des beobachteten Volumens.

Die Art der Beobachtung bzw. Charakterisierung der Fläche oder des Volumens kann dabei reichen von einer einfachen Abbildung, über die Ermittlung von Strukturen bis hin zur physikalischen und/oder chemischen Analyse einzelner Bereiche. Die Charakterisierung kann permanent oder aber in kurzen Abständen während der Lasertherapie des Patientenauges, insbesondere während der Erzeugung der Struktur, erfolgen.

Die erfindungsgemäße Kontrolleinheit wird also demzufolge Bilder und/oder Daten permanent oder in kurzen Abständen empfangen. Dabei werden die Bilder und/oder Daten, die zu einem Startzeitpunkt to, der dem Startzeitpunkt der Erzeugung der Struktur entspricht oder aber zeitlich in nächster Nähe zum Startzeitpunkt der Erzeugung der Struktur liegt, als Referenzbild bzw. Referenzdaten verwendet. Zu diesem Referenzbild bzw. Referenzdaten werden dann die Bilder und/oder Daten, die zu Folgezeitpunkten tᵢ (i= 1 bis n) der Erzeugung der Struktur ermittelt werden, bei der Verarbeitung der Bilder und/oder Daten direkt oder indirekt verglichen.

Weiterhin ist die Kontrolleinheit des erfindungsgemäßen Nachführsystems eingerichtet, eine Position eines Markers in der Fläche oder in dem Volumen des Auges aus den Bildern und/oder Daten der Untersuchungseinheit zu einem Startzeitpunkt to und zu Folgezeitpunkten tᵢ (i= 1 bis n) der Erzeugung der Struktur zu ermitteln.

Ein Marker kann dabei ein natürlicher "Marker" sein, z.B. eine markante Struktur im Auge des Patienten, oder ein künstlich geschaffener Marker. Diese Position des Markers variiert aufgrund der oben beschriebenen unbeabsichtigten Bewegungen des Patientenauges relativ zur ophthalmologischen Lasertherapievorrichtung, und insbesondere zur Nulllage des Laserstrahls, während der ophthalmologischen Lasertherapie. Im erfindungsgemäßen Fall umfasst der Marker jedenfalls den Startpunkt eines beabsichtigten Scanmusters und/oder ist aus einem Algorithmus zur Auswertung des bis zum Zeitpunkt tᵢ vollzogenen Scanmusters erzeugt.

Um die Therapieführung entsprechend dieser Bewegungen des Patientenauges nachzuregeln, ist die Kontrolleinheit des erfindungsgemäßen Nachführsystems weiterhin eingerichtet, aus der Differenz der Position des Markers zum jeweiligen Zeitpunkt tᵢ im Vergleich zum Startzeitpunkt to oder zu einem Zeitpunkt ti-i einen Korrekturparameter K(ti) zu bestimmen, der dazu dient, die Therapieführung zur Erzeugung der Struktur nachzuregeln.

Findet eine Betrachtung der Position des Markers zum Zeitpunkt tᵢ im Vergleich zum Startzeitpunkt to statt, so dienen die Bilder und/oder Daten, die vor oder zu Beginn der Erzeugung der Struktur ermittelt worden, über die gesamte Dauer der Therapieführung als Referenzbild bzw. Referenzdaten. Dies ist vorteilhaft, wenn auch Korrekturen nur von Abschnitten der Therapieführung vorgenommen werden sollen. Findet jedoch eine Betrachtung der Position des Markers zum Zeitpunkt tᵢ im Vergleich zum Zeitpunkt ti-i statt, so treten anschließend die Bilder und/oder Daten, die zum Zeitpunkt tᵢ ermittelt wurden, an die Stelle des Referenzbildes bzw. der Referenzdaten. In diesem Falle muss jedoch immer eine Gesamtkorrektur der Therapieführung, zumindest des noch verbleibenden Teils, vorgenommen werden.

Der zu bestimmende Korrekturparameter K(ti) ist, wenn eine Fläche des Auges beobachtet wurde, ein zweidimensionaler Korrekturparameter, der in der Regel eine Positionskorrektur in der lateralen Ebene bewirkt. Wird hingegen ein Volumen des Auges beobachtet, dann wird daraus ein dreidimensionaler Korrekturparameter bestimmt, so dass eine Reaktion auf Änderungen der relativen Lage des Auges zur ophthalmologischen Lasertherapievorrichtung nicht nur lateral, sondern auch in der z-Richtung bzw. Axialrichtung, die parallel der optischen Achse der ophthalmologischen Lasertherapievorrichtung am Austrittsort des Laserstrahls verläuft, möglich ist.

Die Nachregelung der Therapieführung ist dabei qualitativ umso besser, je kleiner die zeitlichen Abstände tᵢ - ti-i zwischen zwei Positionsbestimmungen des Markers gewählt werden. Geht die Differenz tᵢ - tᵢ₋₁ gegen 0, dann findet eine quasikontinuierliche Nachführung statt.

Unter Einsatz des erfindungsgemäßen Nachführsystems ist somit eine signifikante Verbesserung der Zielformtreue möglich. Ein Scanmuster, das die zeitliche Änderung der Lage eines Fokus der Laserstrahlung, die zur Therapie des Patientenauges verwendet wird, im Gewebe des Auges beschreibt und somit den Ablaufplan der Therapieführung darstellt, wird durch eine Bilderkennung und Bildverfolgung effektiv stabil gehalten. Dies sichert die ortsgenaue und vollständige Ausführung von Strukturierungen, beispielsweise von Schnitten. Somit wird letztlich der Startpunkt des Scanmusters wiedergefunden und eine geschlossene Struktur erzeugt.

Damit zeigt diese Erfindung beispielsweise auch einen Weg, wie die Vollständigkeit des Kapsulotomieschnitts gewährleistet werden kann.

Das erfindungsgemäße Nachführsystem erlaubt die Korrektur unbeabsichtigter Änderungen der relativen Lage von Auge zu System. Es erlaubt, wenn dies gewünscht ist, jedoch auch, eine ophthalmologische Lasertherapievorrichtung ohne eine im Stand der Technik allgemein übliche Fixierung des Patientenauges an das ophthalmologische Lasertherapievorrichtung zu betreiben, ohne dass die Verfahrensdauer der Lasertherapie auf eine Minimalzeit, die kleiner ist als die zeitlichen Abstände so genannter Mikrosakkaden, also unbewusste, schnelle ruckartige Bewegungen des Auges, die nicht durch bewusstes Handeln abstellbar sind, beschränkt ist: Mit seiner Hilfe wird die Therapieführung beständig entsprechend der dann erfolgenden freien Augenbewegungen nachgeregelt.

In einer Ausgestaltung ist das erfindungsgemäße Nachführsystem eingerichtet, auf eine interne Untersuchungseinheit der ophthalmologischen Lasertherapievorrichtung zuzugreifen. Diese interne Untersuchungseinheit der ophthalmologischen Lasertherapievorrichtung muss dazu während der Lasertherapie, also insbesondere während der Erzeugung der Struktur, betreibbar sein.

In einer anderen Ausgestaltung umfasst das erfindungsgemäße Nachführsystem eine Untersuchungseinheit. Diese Ausgestaltung stellt einerseits eine Alternative zu einer Ausgestaltung dar, bei der auf eine interne Untersuchungseinheit der ophthalmologischen Lasertherapievorrichtung zugegriffen wird - etwa wenn die ophthalmologische Lasertherapievorrichtung gar keine Untersuchungseinheit enthält. Andererseits kann bei einem solchen Nachführsystem mit Untersuchungseinheit auch gleichzeitig ein Zugriff auf eine interne Untersuchungseinheit der ophthalmologischen Lasertherapievorrichtung bestehen, wenn die ophthalmologische Lasertherapievorrichtung eine solche eigene Untersuchungseinheit umfasst.

Die Untersuchungseinheit des Nachführsystems muss dazu im Einsatzfall Zugang zum optischen Weg der ophthalmologischen Lasertherapievorrichtung haben, also derart angeordnet sein, dass es das Bearbeitungsvolumen der ophthalmologischen Lasertherapievorrichtung im Patientenauge anvisiert. Zudem darf der optische Weg zwischen der Untersuchungseinheit und dem Auge durch die Therapieführung mittels der ophthalmologische Lasertherapievorrichtung zu keinem Zeitpunkt durch Elemente der Vorrichtung abgeschattet werden.

Der durch das Nachführsystem ermittelte Korrekturparameter kann nun an verschiedenen Stellen eingesetzt werden, um die Therapieführung zur Erzeugung der Struktur nachzuregeln.

Eine Ausführung des erfindungsgemäßen Nachführsystems ist - insbesondere durch eine entsprechende Ausgestaltung der Kontrolleinheit - eingerichtet, mit dem Korrekturparameter K(ti) zum Zeitpunkt tᵢ eine Kalibrierung einer Relativbeziehung von Auge zur ophthalmologischen Lasertherapievorrichtung zu aktualisieren. Dies wird beispielsweise durch direkte Korrektur der Scannerstellungen bzw. der Scannerpositionen erreicht. Im Prinzip wird dadurch eine Nullposition der Scanner, die die Lage des Fokus der Laserstrahlung verändern, beständig neu definiert.

Die Aktualisierung der Kalibrierung einer Relativbeziehung von Auge zur ophthalmologischen Lasertherapievorrichtung erfolgt dabei durch Weitergabe des Korrekturparameter K(ti) an eine Steuereinrichtung oder Planungseinrichtung einer Steuereinrichtung einer ophthalmologischen Lasertherapievorrichtung. Die Abweichungen, die der Korrekturparameter K(ti) beschreibt, führen damit direkt zu neuen Koordinaten an den Scannern. Beispielsweise werden dazu Schrittmotoren einer "Moving Lens" so initiiert, dass durch eine laterale Bewegung dieser eine Nullposition der Moving Lens neu festgelegt wird. Damit wird auch die Umrechnung der Koordinaten des Beobachtungs- bzw. Arbeitsvolumens im Gewebe des Auges in Koordinaten des Scannersystems, also entsprechende Scannerstellungen, jedes Mal aufs Neue verändert.

Diese Ausführung des erfindungsgemäßen Nachführsystems zeichnet sich also aus durch eine direkte Umrechnung der Abweichungen im Koordinatensystem eines Beobachtungsvolumens bzw. des Gewebes des Auges, die durch den Korrekturparameter K(ti) gekennzeichnet sind, in Änderungen der Scannerparameter, bzw. allgemeiner in Änderungen der Parameter einer Einrichtung zur Veränderung der Lage des Fokus der Laserstrahlung.

In einem Nachführsystem, das Bilder und/oder Daten einer Untersuchungseinheit verarbeitet, die eine Fläche des Auges beobachtet, wird die Kalibrierung der Scanner mit einem zweidimensionalen Korrekturparameter K(ti) beaufschlagt werden. Da es sich hier in der Regel um die laterale Ebene, also die x-y-Ebene handelt, die beobachtet wird, wird die Kalibrierung der Scanner mittels einer lateralen Bewegung aktualisiert werden.

In einem Nachführsystem, das Bilder und/oder Daten einer Untersuchungseinheit verarbeitet, die ein Volumen des Auges beobachtet, wird die Kalibrierung der Scanner mit einem dreidimensionalen Korrekturparameter K(ti) beaufschlagt werden. Es wird damit eine komplett räumliche Nachregelung der Therapieführung vollzogen.

Eine Alternative der notwendigen, durch den Korrekturparameter K(ti) qualitativ bestimmten, Korrektur zur Nachregelung einer Therapieführung sieht ein erfindungsgemäßes Nachführsystem vor, das eingerichtet ist, ein Scanmuster, durch das die zeitliche Änderung der Lage eines Fokus der Laserstrahlung im Gewebe des Auges beschrieben ist, zum Zeitpunkt tᵢ mit dem Korrekturparameter K(ti) zu beaufschlagen. Vorteilhaft empfängt hierfür das Nachführsystem, insbesondere dessen Kontrolleinheit, das Scanmuster von der ophthalmologischen Lasertherapievorrichtung, korrigiert es entsprechend und gibt den korrigierten Wert an die ophthalmologische Lasertherapievorrichtung zurück.

Zur Bestimmung der Lage des Fokus der Laserstrahlung wird Bezug genommen auf die drei Raumrichtungen x, y, und z bzw. analoge Koordinatensysteme, wobei die Richtung z zur optischen Achse der ophthalmologischen Lasertherapievorrichtung parallel verläuft und die beiden Richtungen x und y laterale Richtungen darstellen.

Das Scanmuster als ein Ablaufplan der Therapieführung, also beispielsweise als Ablaufplan zur Bildung von Schnittflächen, wird üblicherweise so beschrieben, dass von einer festen Beziehung zwischen der ophthalmologischen Lasertherapievorrichtung und dem Auge des Patienten ausgegangen wird. Auge und Lasertherapievorrichtung werden zueinander kalibriert, danach wird das Scanmuster im Stand der Technik entsprechend dieser Kalibration ausgeführt. Das führt dann zu Fehlern, wenn die als fest angenommene Beziehung zwischen Auge und ophthalmologischer Lasertherapievorrichtung in Realität veränderlich wird.

Einer Änderung der relativen Lage des Patientenauges zur ophthalmologischen Lasertherapievorrichtung zum Zeitpunkt tᵢ im Vergleich zum Startzeitpunkt to bzw. im Vergleich zum Zeitpunkt tᵢ₋₁ wird nun in dieser Korrekturalternative des erfindungsgemäßen Nachführsystems durch Beaufschlagung des Scanmusters, und damit der Abfolge der Fokuspositionen, mit dem Korrekturparameter K(ti) Rechnung getragen.

Dies kann dadurch erfolgen, dass die durch den Korrekturparameter K(ti) gekennzeichneten Abweichungen an eine Steuereinrichtung der ophthalmologischen Lasertherapievorrichtung bzw. eine Planungseinrichtung, die mit der Steuereinrichtung und dem Nachführsystem kommuniziert, weitergegeben werden, das dann das Scanmuster mit dem Korrekturparameter beaufschlagt. Alternativ kann jedoch auch das Scanmuster von der ophthalmologischen Lasertherapievorrichtung empfangen, d.h. an die Kontrolleinheit des Nachführsystems übertragen werden, dort entsprechend verändert werden und anschließend das aktualisierte Scanmuster an die Steuereinrichtung der ophthalmologischen Lasertherapievorrichtung zurückgegeben werden.

Der Empfang bzw. die Übermittlung von Bildern, Daten bzw. entsprechenden Parametern erfolgt dabei zwischen dem Nachführsystem und dem ophthalmologischen Lasertherapievorrichtung, aber auch innerhalb dieser Vorrichtungen über verschiedenste Kommunikationswege, also beispielsweise Kabelverbindungen oder auch kabellose Verbindungen.

In dieser Korrekturalternative des erfindungsgemäßen Nachführsystems muss im Anschluss noch jeweils eine Umrechnung der mit dem Korrekturparameter K(ti) korrigierten Scanmusterpositionen im Koordinatensystem eines Beobachtungsvolumens bzw. des Gewebes des Auges in entsprechende Änderungen der Scannerparameter erfolgen.

In einem Nachführsystem, das Bilder und/oder Daten einer Untersuchungseinheit verarbeitet, die eine Fläche des Auges beobachtet, wird das Scanmuster mit einem zweidimensionalen Korrekturparameter K(ti) beaufschlagt. Damit wird das Scanmuster nur bezüglich Änderungen der relativen Lage des Auges zur ophthalmologischen Lasertherapievorrichtung in einer lateralen Ebene korrigiert. Häufig ist dies jedoch schon ausreichend.

Dabei wird im Prinzip eine Spur verfolgt bzw. korrigiert. Diese Spur ist eine Art makroskopisches Scanmuster. Sie entsteht aus der Aufsicht auf eine Projektionsfläche durch diese Struktur bzw. das mikroskopische Scanmuster. Die Spur ist damit die in dieser Projektionsfläche sichtbare Linie, die während des Scanvorgangs überstrichen wird.

In einem Nachführsystem, das Bilder und/oder Daten einer Untersuchungseinheit verarbeitet, die ein Volumen des Auges beobachtet, wird das Scanmuster mit einem dreidimensionalen Korrekturparameter k(t) beaufschlagt. Jede einzelne Fokusposition des Scanmusters wird dann also bezüglich Änderungen der relativen Lage des Auges zur ophthalmologischen Lasertherapievorrichtung in einem dreidimensionalen Raum korrigiert.

Das Nachführsystem kann dabei in verschiedenen Konstellationen mit der ophthalmologischen Lasertherapievorrichtung zusammenarbeiten. Es kann Teil einer Steuereinrichtung oder einer Planungseinrichtung für die Therapieführung - insbesondere einer Planungseinrichtung für ein Scanmuster zur Erzeugung einer Struktur zur Steuerung einer ophthalmologischen Lasertherapievorrichtung während der ihrer Erzeugung - sein, das eine interne Untersuchungseinheit der ophthalmologischen Lasertherapievorrichtung nutzt. Die Kontrolleinheit des Nachführsystems kann Teil der Steuereinrichtung oder der Planungseinrichtung sein, während das Nachführsystem eine eigene Untersuchungseinheit umfasst. Auch möglich ist es, dass das Nachführsystem eine eigenständige Kontrolleinheit umfasst, die mit der Steuereinrichtung oder der Planungseinrichtung der ophthalmologischen Lasertherapievorrichtung, die wiederum ein Teil der Steuereinrichtung sein kann, kommuniziert, jedoch eine interne Untersuchungseinheit der ophthalmologischen Lasertherapievorrichtung nutzt.

Um die Rechenzeit zu optimieren, kann in einer speziellen Ausgestaltung des erfindungsgemäßen Nachführsystems eine Kontrolleinheit für eine Teilkorrektur des Scanmusters eingerichtet sein: Dabei werden nur die Lagen des Fokus der Laserstrahlung, die in einem Zeitintervall von t > tᵢ bis t ≤ tᵢ₊₁ überstrichen werden, mit dem Korrekturparameter K(ti) beaufschlagt. Hierfür ist es aber notwendig, dass auf ein Referenzbild und/oder Referenzdaten zum Startzeitpunkt to referenziert wird.

Sofern jedoch die Leistung des Nachführsystems nicht limitiert ist, wird bevorzugt das gesamte Muster korrigiert, so dass immer ein Referenz-Scanmuster zum Startzeitpunkt to oder zum Zeitpunkt tᵢ₋₁ und ein aktuell korrigiertes Scanmuster vorhanden ist.

In einer Ausgestaltung ist das erfindungsgemäße Nachführsystem gekennzeichnet durch eine Kontrolleinheit, in die ein Mustererkennungsalgorithmus kodiert ist und/oder die eingerichtet ist zur Erstellung von Differenzbildern. Mit einem Mustererkennungsalgorithmus kann ein vorgegebenes abstraktes Muster in den Bildern und/oder Daten, die die Untersuchungseinheit in einer Fläche oder einem Volumen des Auges ermittelt hat, gefunden werden und/oder ein vor Erzeugung der Struktur gelerntes reales Muster wiedergefunden werden. Durch die Erzeugung von Differenzbildern ist es möglich, Bereiche, in denen eine signifikante Änderung stattfindet, zu kennzeichnen. Die Kontrolleinheit des Nachführsystems kann dabei verschiedene Möglichkeiten bieten, um einen Marker zu finden und zu verfolgen. Diese Möglichkeiten können ggf. auch in Kombination eingesetzt werden - insbesondere, wenn mehrere Untersuchungseinheiten zur Verfügung stehen, die Bilder und/oder Daten zur Charakterisierung einer Fläche oder eines Volumens des Auges liefern können.

Ein entsprechender Marker, dessen Position vom erfindungsgemäßen Nachführsystem erfassbar ist, kann neben dem oben erwähnten Startpunkt eines Scanmusters und/oder Algorithmus zur Auswertung des bis zum Zeitpunkt tᵢ vollzogenen Scanmusters eine natürliche Augenstruktur oder eine künstlich geschaffene Struktur. umfassen. Wenn die Spur eines bereits vollzogenen Teils eines Scanmusters in einem Videobild sichtbar ist, kann diese zur jeweiligen Erzeugung eines Referenzpunkts genutzt werden. Der Marker kann zudem einteilig oder mehrteilig ausgeführt sein: Mehrteilige Marker erlauben häufig, den Korrekturparameter mit größerer Sicherheit zu bestimmen, da ggf. Fehlmessungen bzw. eine fehlerhafte Mustererkennung bzgl. eines Ortes sicherer erkannt werden.

Als Marker, die eine natürliche Augenstruktur umfassen, eignen sich beispielsweise Strukturen der Iris - auch im dilatierten Zustand -, oder Äderchen auf der Netzhaut oder auf der Sklera. Wird eine natürliche Augenstruktur als Marker verwendet, so sollte diese bevorzugt in der Nähe der zu erzeugenden Struktur angeordnet sein.

Das erfindungsgemäße Nachführsystem in seinen verschiedenen Ausführungsformen ist insbesondere vorteilhaft einsetzbar zur Erzeugung von geschlossenen Strukturen, wo der Erfolg der Lasertherapie vom Wiederfinden des Startpunktes des Scanmusters zur Erzeugung dieser geschlossenen Struktur abhängig ist.

Das vom Fokus der Laserstrahlung abzufahrende Scanmuster ist dabei in allen Ausführungsformen optional hinterlegt in einer Auswahltabelle von Scanmustern in einer Planungseinrichtung, die mit der Steuereinrichtung des ophthalmologischen Lasertherapiesystems zusammenarbeitet, oder direkt in der Steuereinrichtung. Alternativ oder aber zur gleichzeitigen Nutzung ist ein Algorithmus zur Erstellung eines Scanmusters der Struktur in einer Planungseinrichtung oder Steuereinrichtung kodiert.

Wenn eine Fläche des Auges beobachtet und analysiert wird, also im Falle einer zweidimensionalen Beobachtung des Scanmusters und damit einer Spur eines Scanmusters, dann wird, wie schon erwähnt, üblicherweise auch nur zweidimensional korrigiert, in der Regel also in lateralen Richtungen. Bei einer dreidimensionalen Beobachtung und Korrektur wird auch die Höhe nachgeführt.

Das erfindungsgemäße Nachführsystem ist dabei von Vorteil für alle Scanverfahren wie oben vorgestellt, also in einem Single-Path-Verfahren wie auch in einem Multi-Path-Verfahren. Aber insbesondere die Ausführung geschlossener Strukturen in einem Single-Path-Verfahren, wie beispielsweise ein Kapsulotomie-Schnitt, profitiert von einer Nutzung dieses Nachführsystems.

Eine ophthalmologische Lasertherapievorrichtung umfasst eine Einrichtung zur Erzeugung einer Laserstrahlung, also eine Laserquelle, die einen gerichteten Laserstrahl erzeugt. Die ophthalmologische Lasertherapievorrichtung umfasst weiterhin eine Optik zur Fokussierung der Laserstrahlung in einem Fokus in einem Bearbeitungsvolumen, also in einem dreidimensionalen Bereich der in der Nähe des Austrittsorts der fokussierten Laserstrahlung aus der ophthalmologischen Lasertherapievorrichtung angeordnet ist, und in dem insbesondere ein Patientenauge so anordenbar ist, dass ein Gewebe des Patientenauge in diesem Bearbeitungsvolumen bearbeitet werden kann. Dieses Bearbeitungsvolumen ist mit den drei Raumrichtungen x, y und z beschreibbar, wobei die z-Richtung parallel zur optischen Achse am Austrittsort der fokussierten Laserstrahlung ist, während die xund y-Richtungen lateral verlaufen.

Des Weiteren umfasst die ophthalmologische Lasertherapievorrichtung eine Einrichtung zur Veränderung der Lage des Fokus im Bearbeitungsvolumen. Hierfür sind verschiedenste Scannersysteme einsetzbar: Lateral können zwei voneinander unabhängige Scanner für die Veränderung in x- und y-Richtung eingesetzt werden, oder ein x-y-Scansystem, wie beispielsweise einen R-Scanner, der um die optische Achse rotiert werden kann. Auch ist eine bewegliche Linse einsetzbar, die durch eine langsame laterale x-y-Bewegung den Laserfokus lateral bewegt. Zudem kann zusätzlich zu einem langsamen lateralen Scansystem, das die gesamte x-y-Ausdehnung abdeckt, ein schnelles laterales Teilscansystem eingesetzt werden, das beispielsweise eine schnelle oszillierende Bewegung in der x-y-Ebene ausführt. In z-Richtung hingegen kann ein langsamer z-Scanner durch eine entlang der z-Richtung frei bewegliche Linse ausgeführt sein. Auf diese Bewegung kann zusätzlich ein schneller z-Scanner eine schnelle oszillierende Bewegung in z-Richtung ausführen. Ein solcher schneller z-Scanner kann durch ein kleines, leichtes Linsenelement im Strahlengang der Laserstrahlung, dort wo dieser einen sehr kleinen Durchmesser hat, umgesetzt werden. Die Oszillation kann mittels resonanzbetriebener Voice Coil erfolgen.

Die Optik zur Fokussierung wie auch Elemente der Einrichtung zur Veränderung der Lage des Fokus sind im optischen Weg der Laserstrahlung angeordnet, über den diese von der Einrichtung zur Erzeugung der Laserstrahlung bis ins Bearbeitungsvolumen, in dem im Einsatz ein Gewebe eines Patientenauges angeordnet ist, geformt und gelenkt wird.

Schließlich umfasst die ophthalmologische Lasertherapievorrichtung eine Steuereinrichtung zur Steuerung der Lasertherapievorrichtung. Diese Steuereinrichtung kann auf die steuerbaren Einrichtungen der Lasertherapievorrichtung zugreifen, also beispielsweise die Einrichtung zur Erzeugung der Strahlung, die Einrichtung zur Veränderung der Lage des Fokus und ggf. auch die Optik zur Fokussierung, um diese zu steuern. Die Steuereinrichtung kann eine Planungseinrichtung bzw. Planungseinheit zur Erstellung des Scanmusters, also des zeitlichen Verlaufs der Fokusführung während der Lasertherapie, enthalten, oder aber auf eine solche externe Planungseinrichtung zugreifen. Zumeist enthält sie außer den Koordinateninformationen auch Informationen zu zeitlich bzw. örtlich abhängigen Parametern der Steuerung der Einrichtung zur Erzeugung der Laserstrahlung, in Summe also die Informationen zur Therapieführung. Die Steuereinrichtung ist mit den steuerbaren Einrichtungen der ophthalmologischen Lasertherapievorrichtung wie auch ggf. mit der Planungseinrichtung über Kommunikationswege, d.h. Drahtverbindungen bzw. drahtlose Verbindungen verbunden.

Die erfindungsgemäße ophthalmologische Lasertherapievorrichtung ist nun dadurch gekennzeichnet, dass sie weiterhin ein Nachführsystem zur Nachregelung der Therapieführung enthält wie es oben beschrieben wurde. Das Nachführsystem erlaubt die aktive Korrektur unbeabsichtigter Änderungen einer relativen Lage vom Patientenauge zum ophthalmologischen Lasertherapiesystem und hier insbesondere zu einer Nulllage der zur Therapie eingesetzten, gerichteten Laserstrahlung. Dies führt zu einer beständigen Kontrolle und Korrektur der Ausrichtung von Patientenauge und ophthalmologischen Lasertherapiesystem zueinander bzw. zu einer Berücksichtigung von Änderungen der relativen Lage von Patientenauge und ophthalmologischen Lasertherapiesystem zueinander unmittelbar bei der Therapieführung durch Aktualisierung eines Scanmusters im Verlaufe der Lasertherapie.

Damit erlaubt die erfindungsgemäße ophthalmologische Lasertherapievorrichtung, auf Augenbewegungen während der Lasertherapie aktiv - in zeitlich sehr kurzen Abständen oder aber quasi-kontinuierlich - zu reagieren und einen entsprechenden Versatz zu vermeiden. Insbesondere bei der Erzeugung von geschlossenen Strukturen in einem Augengewebe, bei denen ein Endpunkt eines Scanmusters den Startpunkt wieder erreichen sollte, werden dadurch Komplikationen vermieden. Aber auch offene Strukturen können örtlich wesentlich genauer in einem Augengewebe erzeugt werden. Eine Fehlsichtigkeitskorrektur ist dann beispielsweise mit höherer Präzision möglich. Der wichtigste Anwendungsbereich einer solchen erfindungsgemäßen Lasertherapievorrichtung ist dabei die Ausführung von Schnitten in einem Augengewebe: Die Therapieführung ist in diesem Fall dann eigentlich eine Schnittführung.

Die Grundidee der Erfindung basiert also auf dem Einsatz eines Nachführsystems, auch Eyetracker genannt, das die Bewegung des Patientenauges verfolgt und die Therapieführung, insbesondere eine Schnittführung, aktiv nachregelt.

In einer besonderen Ausgestaltung der ophthalmologische Lasertherapievorrichtung enthält diese eine Einrichtung zur Erzeugung einer Laserstrahlung, die eine gepulste Laserstrahlung, insbesondere eine Femtosekunden-Laserstrahlung, erzeugt.

In einer bevorzugten Ausgestaltung enthält die ophthalmologische Lasertherapievorrichtung ein Patienteninterface, insbesondere ein Flüssigkeitspatienteninterface oder ein Kontaktglas.

In einer anderen bevorzugten Ausgestaltung umfasst die ophthalmologische Lasertherapievorrichtung eine interne Untersuchungseinheit zur Charakterisierung des Auges, das entsprechende Bilder und/oder Daten zur einer Fläche oder eines Volumens des Auges erzeugt, sowie ein oben beschriebenes Nachführsystem. In diesem Fall kann das Nachführsystem auf die Bilder und/oder Daten der internen Untersuchungseinheit der ophthalmologischen Lasertherapievorrichtung zurückgreifen. Sofern das Nachführsystem aber auch eine eigene Untersuchungseinheit aufweist, kann wahlweise die interne Untersuchungseinheit der ophthalmologischen Lasertherapievorrichtung als auch die Untersuchungseinheit des Nachführsystems zur Charakterisierung einer Fläche oder eines Volumens des Patientenauges eingesetzt werden, um entsprechende Bilder und/oder Daten für das Nachführsystem zu erzeugen.

In weiteren Ausgestaltungen des erfindungsgemäßen Nachführsystems, das eingerichtet ist zur Verarbeitung von Bildern und/oder Daten einer Untersuchungseinheit, umfasst die Untersuchungseinheit mindestens eine der folgenden Untersuchungseinrichtungen: ein Videosystem, eine optische Kohärenztomographie (OCT)-Vorrichtung, insbesondere ein live-OCT, eine Ultraschallmessvorrichtung, einen konfokalen Detektor und/oder eine Scheimpflug-Kamera. Für verschiedene Anwendungen können dabei zur Nachregelung der entsprechenden Therapieführung ggf. auch verschiedene Untersuchungseinrichtungen verwendet werden.

Besonders vorteilhaft ist dabei der Einsatz einer Videokamera, die ein Videobild in Draufsicht erzeugt und in der Regel eine zweidimensionale, hier laterale Nachregelung der Therapieführung erlaubt. Diese Videokamera kann entweder im sichtbaren Spektralbereich oder im Infrarotbereich arbeiten, wobei ein Arbeiten im Infrarotbereich deshalb vorteilhaft ist, weil in einem Infrarotbild selbst bei einer dunklen Iris detaillierte Strukturen erkennbar sind.

Für die Charakterisierung des Volumens des Auges zur Bestimmung der Position eines Markers und die anschließende dreidimensionale Nachregelung der Therapieführung ist hingegen eine OCT-Vorrichtung besonders vorteilhaft.

In einem Verfahren zur Nachregelung einer Therapieführung einer ophthalmologischen Lasertherapievorrichtung zur Erzeugung einer Struktur in einem Gewebe eines Auges bzw. generell in einem Bearbeitungsvolumen der ophthalmologischen Lasertherapievorrichtung, welches explizit nicht Teil der Erfindung ist und nur als Beispiel einer Anwendung beschrieben wird, werden
- Bilder und/oder Daten einer Untersuchungseinheit, die eine Fläche oder ein Volumen des Auges beobachtet, empfangen und verarbeitet,
- aus den Bildern und/oder Daten der Untersuchungseinheit zu einem Startzeitpunkt to und zu Folgezeitpunkten tᵢ (i= 1 bis n) der Erzeugung der Struktur eine Position eines Markers, der einen Startpunkt eines Scanmusters umfasst, und/oder aus einem Algorithmus zur Auswertung des bis zum Zeitpunkt tᵢ vollzogenen Scanmusters erzeugt wird in der Fläche oder in dem Volumen des Auges ermittelt, und es wird
- aus der Differenz der Position des Markers zum Zeitpunkt tᵢ im Vergleich zum Startzeitpunkt to oder zu einem Zeitpunkt tᵢ₋₁ ein Korrekturparameter K(ti) bestimmt. Letztlich wird damit der Startpunkt des Scanmusters wiedergefunden, um eine geschlossene Struktur zu erzeugen.

Die Bilder bzw. Daten der Untersuchungseinheit zeigen dabei beispielsweise Strukturen, Grenzflächen und/oder unterschiedliche Zusammensetzungen des Materials des Augengewebes bzw. einer Abfolge von Augengeweben auf.

Die Beobachtung der Fläche oder des Volumens des Auges erfolgt über eine entsprechend große Fläche bzw. über ein großes Volumen, sodass sämtliche Relativbewegungen des Auges, ausgelöst durch natürliche Augenbewegungen, Atembewegungen, aber auch anderer Änderungen der relativen Lage des Auges zum ophthalmologischen Lasertherapiesystem dabei erfasst werden können. Ein Durchmesser der beobachteten Fläche oder des beobachteten Volumens sollte also signifikant größer sein als der größte zu erwartende Versatz aufgrund von Bewegungen des Patientenauges relativ zur ophthalmologischen Lasertherapievorrichtung während einer ophthalmologischen Lasertherapie, durch die sich die Position des Markers verändert.

Die Beobachtung der Fläche oder des Volumens und insbesondere der Empfang und die Verarbeitung der Daten zum Zwecke der Bestimmung eines Korrekturparameters erfolgt permanent oder in kurzen Abständen zum Startzeitpunkt to und zu Folgezeitpunkten tᵢ. Diese zeitlichen Abstände können auch variieren, so dass in kritischen Bereichen der Therapieführung eine Nachregelung in kürzeren Abständen erfolgt als in unkritischen Bereichen der Therapieführung.

Der Marker, dessen Position in der von der Untersuchungseinheit beobachteten Fläche oder dem beobachteten Volumen ermittelt wird, kann ein natürlicher Marker sein, z.B. eine markante Struktur im Auge, oder ein künstlich geschaffener Marker oder aber ein Teil des schon im Gewebe des Auges vollzogenen Scanmusters, also des bereits erzeugten Teils der Struktur.

Es wird ein zweidimensionaler Korrekturparameter K(ti) bestimmt, wenn Bilder und/oder Daten einer durch die Untersuchungseinheit beobachteten Fläche zu dessen Ermittlung verwendet werden. Wird hingegen durch die Untersuchungseinheit ein Volumen beobachtet und entsprechende dreidimensionale Bilder und/oder Daten erzeugt, so kann daraus ein dreidimensionaler Korrekturparameter K(ti) bestimmt werden.

Mit dem Korrekturparameter K(tᵢ) wird zum Zeitpunkt tᵢ eine Kalibrierung der Relativbeziehung von Auge und ophthalmologischer Lasertherapievorrichtung, also deren relativer Lage zueinander, aktualisiert. Dies kann durch direkte Korrektur in der Einrichtung zur Veränderung der Lage, also beispielsweise direkte Korrektur der Scanner, in der Regel unter Zuhilfenahme der Steuereinrichtung erfolgen.

Alternativ kann ein Scanmuster, das die zeitliche Änderung der Lage eines Fokus einer Laserstrahlung der ophthalmologischen Lasertherapievorrichtung im Gewebe des Patientenauges, beispielsweise bezugnehmend auf die drei Raumrichtungen x, y, und z eines Bearbeitungsvolumens, in dem das Patientenauge angeordnet wird, beschreibt, zum Zeitpunkt tᵢ mit dem Korrekturparameter K(ti) beaufschlagt werden. Dafür kann ein Nachführsystem das Scanmuster von der ophthalmologischen Lasertherapievorrichtung empfangen, entsprechend korrigieren und das mit dem Korrekturparameter K(ti) beaufschlagte Scanmuster an die ophthalmologische Lasertherapievorrichtung, insbesondere an eine Steuereinrichtung der ophthalmologischen Lasertherapievorrichtung, bevorzugt an eine Planungseinrichtung der Steuereinrichtung, auf die die Steuereinrichtung im Verlauf der Lasertherapie permanent oder in kurzen Zeitabständen zugreift, zurückgegeben.

Andererseits kann auch der Korrekturparameter K(ti) an eine Steuereinrichtung oder an eine Planungseinrichtung der Steuereinrichtung der ophthalmologischen Lasertherapievorrichtung übertragen werden und dort zur Nachregelung verwendet werden.

Die vorliegende Erfindung soll nun anhand von Ausführungsbeispielen erläutert werden. Da die Kapsulotomie, also die anteriore Öffnung des Kapselsacks, wie sie für eine Katarakt-Operation genutzt wird, um im weiteren Verlauf durch diese Öffnung hindurch eine fragmentierte Linse des Auges zu evakuieren, ein sensibler Schritt ist, wird der Kapsulotomie-Schnitt mit Hilfe einer fokussierten Laserstrahlung zur Erläuterung verschiedener Ausführungsformen der Erfindung genutzt. Diese sind jedoch auch auf andere zu erzeugende Strukturen im Auge übertragbar. Es zeigt:
- die Fig. 1a und 1b: einen Kapsolutomie-Schnitt nach dem Stand der Technik nach dem Multi-Path-Verfahren und ein dabei auftretender Versatz durch Bewegungen des Patientenauges wie oben beschrieben.
- die Fig. 2a und 2b: einen Kapsolutomie-Schnitt nach dem Stand der Technik nach dem Single-Path-Verfahren und ein dabei auftretender Versatz durch Bewegungen des Patientenauges wie oben beschrieben.
- die Fig. 3a und 3b: ein gegenüber dem der Fig. 2a und 2b vorteilhaft weiterentwickeltes Laserschnitt-Führungskonzept nach dem Single-Path-Verfahren wie oben beschrieben.
- die Fig. 4: eine offene Spur einer Schnittkurve einer Kapsulotomie ohne Nachregelung der Therapieführung.
- die Fig. 5: die geschlossene Spur einer Schnittkurve einer Kapsulotomie mit Nachregelung der Therapieführung.
- die Fig. 6: einen ersten Teil der Spur einer Schnittkurve einer Kapsulotomie mit einem ersten Ausführungsbeispiel der Nachregelung der Therapieführung.
- die Fig. 7: einen ersten Teil der Spur einer Schnittkurve einer Kapsulotomie mit einem zweiten Ausführungsbeispiel der Nachregelung der Therapieführung.
- die Fig. 8: ein erstes Ausführungsbeispiel einer erfindungsgemäßen ophthalmologischen Lasertherapievorrichtung
- die Fig. 9: ein zweites Ausführungsbeispiel einer erfindungsgemäßen ophthalmologischen Lasertherapievorrichtung
- die Fig. 10: ein drittes Ausführungsbeispiel einer erfindungsgemäßen ophthalmologischen Lasertherapievorrichtung

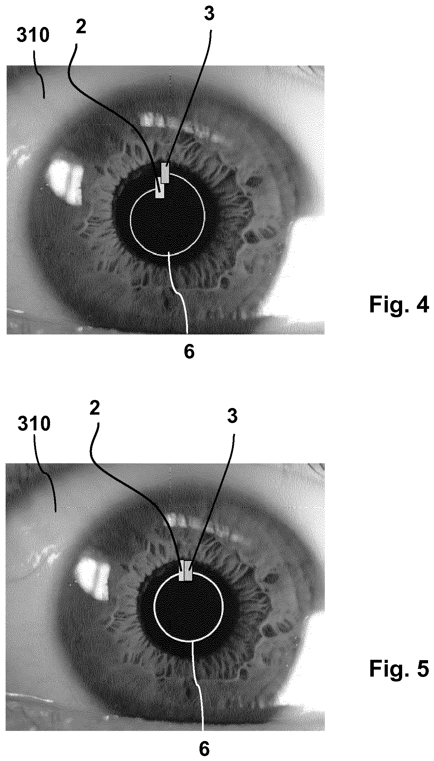

Die Fig. 4 zeigt eine offene Spur 6 einer Kapsulotomie-Schnittkurve, bei der die Therapieführung nicht nachgeregelt wurde, obwohl sich das Patientenauge 310 während des Schneideprozesses bewegt hat, und auch keine weiteren Maßnahmen getroffen wurden, um eine Überlappung von Startpunkt bzw. Startbereich 2 und Endpunkt bzw. Endbereich 3 der Spur 6 zu ermöglichen. Durch die Bewegung des Patientenauges 310 während der Therapieführung wurde die Schnittkurve der Kapsulotomie also nicht geschlossen und damit das Kapsulotomie-Häutchen nicht vollständig abgetrennt. Soll das Kapulotomie-Häutchen entfernt werden, muss es abgerissen werden, was die Schnittkante weiter destabilisiert und in den der Kapsulotomie folgenden Schritten zu Komplikationen führen kann.

Die Fig. 5 zeigt hingegen eine Spur 6 einer Kapsulotomie-Schnittkurve mit Nachregelung der Therapieführung mittels eines erfindungsgemäßen Nachführsystems.

Die Spur 6 ist im Gegensatz zu jener in der Fig. 4 geschlossen, obwohl sich auch hier das Auge 310 während des Schneideprozesses, der durch Photodisruption mit einer Femtosekunden-Laserstrahlung 110 erfolgt, bewegt hat.

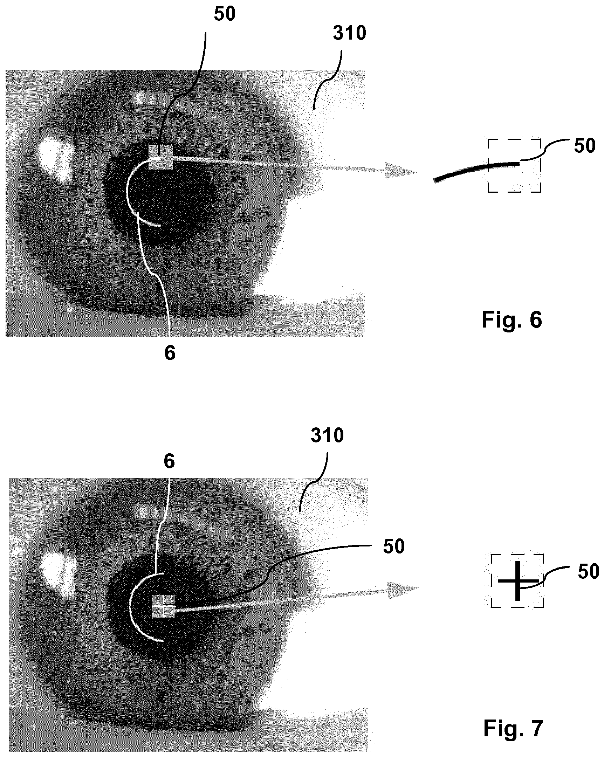

In der Fig. 6 ist ein erster Teil einer Spur 6 einer Kapsulotomie-Schnittkurve mit einem ersten Ausführungsbeispiel der Nachregelung der Therapieführung dargestellt. In diesem Beispiel wird der Startpunkt der Kapsulotomie als der zu verfolgende Marker 50 genutzt.

Das "Schneiden" des Lasers in den jeweiligen Fokuswirkbereichen 8 hinterlässt eine Spur 6 im Linsen- bzw. Kapselsackgewebe des Auges 310. Diese Spur 6 kann man mit einer Videokamera 660 in einer Draufsicht erkennen. Der Startpunkt 2 dieser Spur 6 eignet sich hierbei als zu verfolgender Marker 50 bei Einsatz eines Nachführsystems 700, auch Eyetracking genannt, das als Untersuchungseinheit 600 eine Videokamera 660 nutzt. Der Startpunkt 2 des Kapsulotomieschnitts wird dabei durch Bildverarbeitung der Bilder der Videokamera 660 registriert und während der Erzeugung des Kapsulotomieschnitts aktiv verfolgt. Eine Bewegung dieses Startpunkts 2 bedeutet automatisch eine Bewegung des Auges 310, und damit eine Änderung der relativen Lage des Auges 310 zur ophthalmologischen Lasertherapievorrichtung 800, die verfolgt wird. Die ermittelten Abweichungen zur Lage des Markers 50 zum Startzeitpunkt to werden als Korrekturparameter K(ti) - in diesem Fall zweidimensionale Korrekturparameter K(ti) - zur aktiven Nachführung der Schnittkurve, d.h. zur Nachführung der einzelnen noch verbleibenden Fokuspositionen 4 des Scanmusters 140, genutzt. Damit erreicht der Endpunkt 3 der Schnittkurve 1 im Schnittverlauf den Startpunkt 2 wieder, die sich dann genau, wie in Fig. 5 gezeigt, überlagern.

Mit diesem Lösungsansatz wird sichergestellt, dass die Kapsulotomie komplett durchgeschnitten ist: Es gibt also keine verbleibenden Hautstege des Kapsulotomie-Häutchens. Damit wird auch das Risiko eines Kapselrisses geringer. Außerdem wird die Schnitt-Zirkularität verbessert. Ohne aktive Nachregelung der Schnittführung würde jede Augenbewegung zu einer Modulation auf der ideal kreisrunden Schnittlinie führen. Mit aktiver Nachregelung der Schnittführung mittels des erfindungsgemäßen Nachführsystems, auch Eyetracker genannt, bzw. mit der Schnittführung des Kapsulotomieschnitts - werden die Modulationen kleiner und gehen bei einer beliebig schnellen Nachführung gegen Null.

Die Fig. 7 zeigt einen ersten Teil einer Spur 6 einer Kapsulotomie-Schnittkurve mit einem zweiten Ausführungsbeispiel der Nachregelung der Therapieführung. In diesem Beispiel wird als zu verfolgender Marker 50 vor Beginn des eigentlichen Kapsulotomieschnitts ein Kreuzschnitt in den Mittelpunkt der zirkular zu schneidenden Kapsulotomie gesetzt. Alternativ können auch andere Muster, z.B. sternförmige, eckige oder ovale Muster, als Marker 50 verwendet werden, die sich zur Feststellung einer eindeutigen lateralen Lage eignen. Einen nicht zur eigentlich zu erzeugenden Struktur der Kapsulotomie 1 zugehörigen Marker 50 im Mittelpunkt der Kapsulotomie zu setzen ist unkritisch, da dieser Teil nach vollständiger Kapsulotomie sowieso entfernt wird. Dieser Kreuzschnitt-Marker 50 wird von einer Untersuchungseinheit 600, die hier wiederum eine Videokamera 660 ist, während der Kapsulotomie verfolgt. Die entsprechende Position dieses Markers 50 wird zum Startzeitpunkt to und in regelmäßigen, kurz aufeinanderfolgenden Folgezeitpunkten tᵢ, üblicherweise in Abständen im Bereich von 0,1 ms bis 10 ms für kritische Bereiche der Therapieführung und von 1 ms bis 100 ms für unkritische Bereiche der Therapieführung, in diesem konkreten Falle im Abstand von 1 ms für kritische Bereiche der Therapieführung und im Abstand von 50 ms für unkritische Bereiche der Therapieführung, aus den von der Videokamera 660 gelieferten Bildern bzw. gleich den Bilddaten des Markers 50 vom Nachführsystem 700 ermittelt und ein Korrekturparameter K(ti) zum jeweiligen Zeitpunkt tᵢ bestimmt. Mit diesem Korrekturparameter K(tᵢ), der die lateralen Bewegungen des Auges 310 bzw. der relativen Lage des Auges 310 zur ophthalmologischen Lasertherapievorrichtung 800 beschreibt, wird die Therapieführung in diesem Beispiel wahlweise durch den Benutzer auswählbar oder vor Übergabe an den Benutzer festlegbar dadurch nachgeregelt, dass das Scanmuster des Kapsulotomieschnitts in lateraler Richtung korrigiert wird, oder dass die Kalibrierung der Relativbeziehung von Auge 310 zur ophthalmologischen Lasertherapievorrichtung 800 aktualisiert wird, also direkt die Position des x-y-Scanners 400, 411, 412 der ophthalmologischen Lasertherapievorrichtung 800 korrigiert wird.

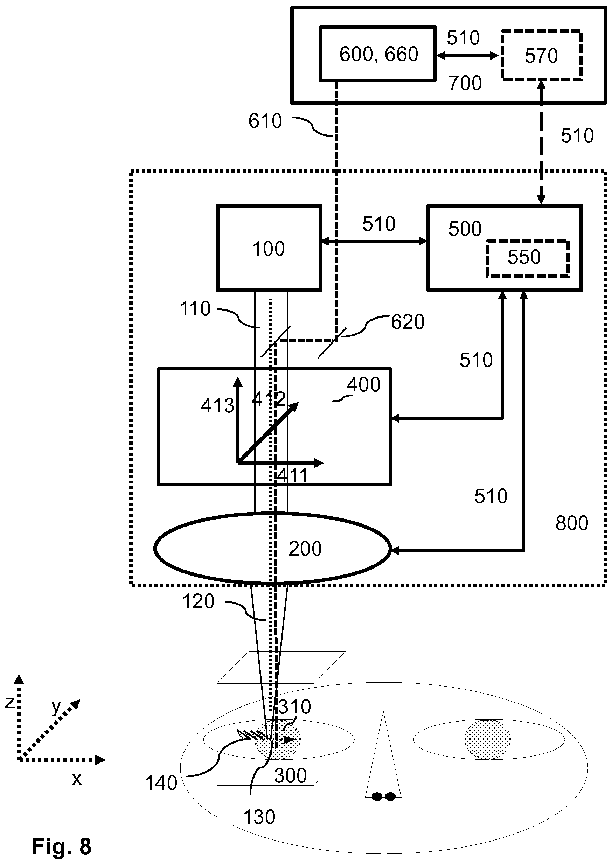

Die Fig. 8 zeigt ein erstes Ausführungsbeispiel einer erfindungsgemäßen ophthalmologischen Lasertherapievorrichtung 800 mit einem Nachführsystem 700.

Die ophthalmologische Lasertherapievorrichtung 800 enthält eine Einrichtung zur Erzeugung 100 einer Laserstrahlung 110, die einen Femtosekunden-Laser mit einer Wellenlänge im Bereich von 1020 nm - 1060 nm umfasst. Die Pulsdauer dieses Femtosekunden-Lasers beträgt 400 - 900 fs, die Pulsenergie ca. 10 µJ.

Des Weiteren enthält die Lasertherapievorrichtung eine Optik 200 zur Fokussierung der Laserstrahlung in einem Fokus 130, mit einer numerischen Apertur von 0,2, die eine Bildfeldgröße von ca. 6mm aufweist. Mit dieser Anordnung ist ein Bearbeitungsvolumen 300 von 6mm x 6mm x 6mm erreichbar, in dem ein Ausschnitt eines Patientenauges 310 platzierbar ist, dessen Gewebe mit Hilfe der fokussierten Laserstrahlung 110 des Femtosekunden-Lasers bearbeitet werden soll. Die optische Achse 120 verläuft parallel zur z-Richtung.

Die ophthalmologische Lasertherapievorrichtung 800 enthält des Weiteren eine Einrichtung 400 zur Veränderung der Lage des Fokus 130 mit einem Scansystem aus drei Scannern 411, 412, 413, die in x-, y bzw. z-Richtung Scanbewegungen ausführen können und durch die Zusammensetzung dieser Bewegungen die Fokusposition der Laserstrahlung 110 in beliebiger Richtung verändern können. Das Scansystem umfasst dabei vorteilhaft ein schnelles Scansystem mit drei schnellen Scannern, die in x-, y- oder z-Richtung schnelle Scanbewegungen ausführen können, und einem langsamen Scansystem mit drei langsamen Scannern, die in x-, y- oder z-Richtung langsame Scanbewegungen ausführen können (in der Figur nicht gezeigt). Ein Scanmuster 140 kann durch die Zusammenarbeit des schnellen und des langsamen Scansystems abgefahren werden.

Des Weiteren enthält die ophthalmologische Lasertherapievorrichtung 800 eine einteilige zentrale Steuereinrichtung 500, die über Kommunikationswege 510, die teilweise drahtlos, teilweise drahtgebunden verlaufen, mit der Einrichtung zur Erzeugung 100 einer Laserstrahlung, also dem Femtosekunden-Lasersystem, und mit der Einrichtung 400 zur Veränderung der Lage des Fokus 130 verbunden ist, und eingerichtet ist, sowohl das Femtosekunden-Lasersystem als auch alle Scanner 411, 412, 413 der Einrichtung 400 zur Veränderung der Lage des Fokus 130 sowie ggf. die Optik 200 zu steuern.

In diesem ersten Ausführungsbeispiel der erfindungsgemäßen ophthalmologischen Lasertherapievorrichtung 800 umfasst die zentrale Steuereinrichtung 500 eine räumlich nicht von der Steuereinrichtung 500 getrennte Planungseinrichtung 550. Die Planungseinrichtung 550 enthält eine Auswahltabelle von Scanmustern 140 von zu erzeugenden Strukturen 1 in den Geweben eines Auges 310. Diese können allgemein entweder nach manueller Bewertung von Charakterisierungsdaten des zu therapierenden Auges 310 durch den Benutzer ausgewählt und gestartet werden, oder aber durch Übertragung von externen Charakterisierungsdaten des Auges 310 an die Steuereinrichtung oder direkt an die Planungseinrichtung und deren Nutzung zur Optimierung dieser Standard-Scanmuster 140 zunächst für das konkrete zu therapierende Auge 310 optimiert und schließlich gestartet werden. Der Start des Scanmusters, und damit die Therapieführung, erfolgt in diesem Fall, indem das entsprechende Scanmuster 140 von der Planungseinrichtung 550 an den allgemeinen Teil der Steuereinrichtung 500 übergeben wird. Zum Startzeitpunkt enthält damit die Steuereinrichtung 500 das entsprechend abzufahrende Scanmuster 140 für die Erzeugung einer Struktur 1 zum Einsatz in einem Gewebe des Patientenauges 310.

Die ophthalmologische Lasertherapievorrichtung 800, und hier insbesondere die Steuereinrichtung 500 der ophthalmologischen Lasertherapievorrichtung 800, kommuniziert in diesem ersten Ausführungsbeispiel mit einem externen Nachführsystem 700, das eine Kontrolleinheit 570 enthält, die über kabellose Kommunikationswege 510 mit der Steuereinheit 500 der ophthalmologischen Lasertherapievorrichtung 800 verbunden ist. Die Kontrolleinheit 570 des Nachführsystems 700 ist eingerichtet, Bilder und Daten einer Untersuchungseinheit 600, hier einer Videokamera 660, zur Charakterisierung einer Fläche des Auges 310 zu empfangen und zu verarbeiten, um eine Position eines Markers 50 in der Fläche des Auges 310 aus den Bildern und Daten der Untersuchungseinheit 600, 660 zu einem Startzeitpunkt to und zu Folgezeitpunkten tᵢ (i= 1 bis n) der Erzeugung der Struktur 1 zu ermitteln, und aus der Differenz der Position des Markers 50 zum jeweiligen Zeitpunkt tᵢ im Vergleich zum Startzeitpunkt to einen Korrekturparameter K(ti) zu bestimmen. Dieses Nachführsystem 700 enthält zu diesem Zwecke neben der Kontrolleinheit 570 eine Videokamera 600, 660, die so angeordnet ist, dass sie eine Fläche des Auges kontinuierlich beobachten kann. Hierbei sind mehrere Ausgestaltungen möglich: Die Videokamera 600, 660 des Nachführsystems 700 kann direkt auf das Auge 310 gerichtet sein. Die Videokamera 600, 660 kann aber auch so an der ophthalmologischen Lasertherapievorrichtung 800 angeordnet sein, dass sie optische Wege der ophthalmologischen Lasertherapievorrichtung 800 nutzt. In allen Fällen ist die Position der Videokamera 600, 660 des Nachführsystems 700 zur ophthalmologischen Lasertherapievorrichtung 800 definiert: Wenngleich die ophthalmologische Lasertherapievorrichtung 800 und das Nachführsystem 700 räumlich voneinander abgegrenzt sind, bilden sie eine Einheit.

Das Nachführsystem 700 gibt den ermittelten, in diesem Fall zweidimensionalen, Korrekturparameter K(ti) an die Steuereinrichtung 500 der ophthalmologischen Lasertherapievorrichtung 800 weiter, die damit die Kalibrierung durch direkte Änderung der Scannerpositionen aktualisiert. Die Scannerpositionen sind eingestellt, um eine gewünschte Position des Fokus 130 im Gewebe des Auges 310 zu erzielen, d.h., es wird ein entsprechender Versatz auf die Scannerpositionen gegeben. Das Scanmuster 140 zur Erzeugung der Struktur 1 wird jedoch mit den ursprünglichen Koordinaten der Fokuspositionen weiter abgearbeitet.

Durch Bild- bzw. Mustererkennung und -verfolgung wird also die Position des Patientenauges 310 lateral effektiv stabil gehalten und damit der Kapsulotomieschnitt 1 zirkular und geschlossen ausgeführt, so dass der Endpunkt 3 des Schnitts mit dem Startpunkt 2 übereinander fällt.

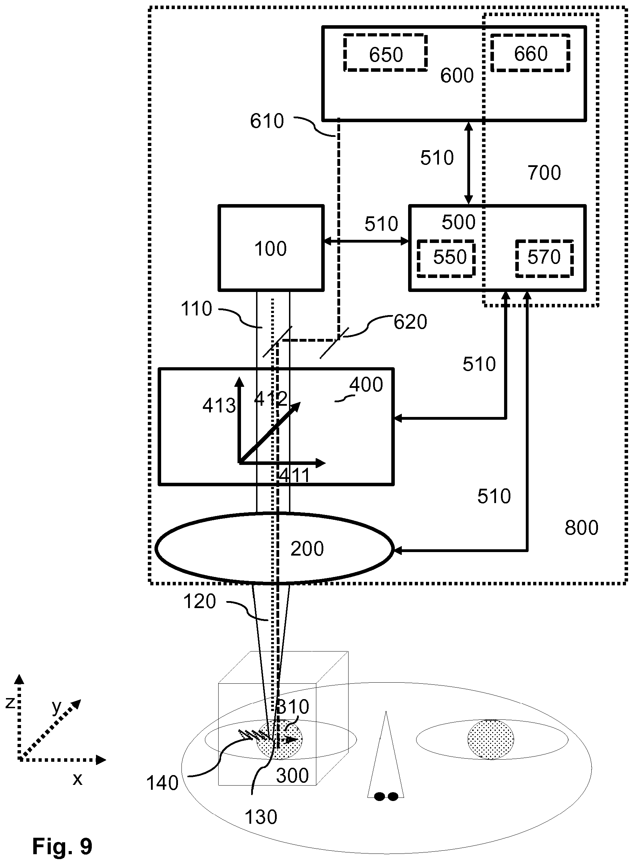

Die Fig. 9 zeigt ein zweites Ausführungsbeispiel einer erfindungsgemäßen ophthalmologischen Lasertherapievorrichtung 800. Während das erste Ausführungsbeispiel eine ophthalmologische Lasertherapievorrichtung mit einem separaten Nachführsystem 700 beschreibt, enthält dieses zweite Ausführungsbeispiel der erfindungsgemäßen ophthalmologischen Lasertherapievorrichtung 800 ein komplett integriertes Nachführsystem 700.

Die Einrichtung zur Erzeugung 100 einer Laserstrahlung, die Optik 200 zur Fokussierung der Laserstrahlung in einem Fokus 130 und die Einrichtung 400 zur Veränderung der Lage des Fokus 130 entsprechen prinzipiell denen des in der Fig. 8 beschriebenen ersten Ausführungsbeispiels.

Dieses zweite Ausführungsbeispiel einer ophthalmologischen Lasertherapievorrichtung 800 enthält weiterhin eine Untersuchungseinheit 600 mit zwei Untersuchungseinrichtungen: eine Videokamera 660 und eine optische Kohärenztomographie (OCT)-Vorrichtung 650. Beide sind so eingerichtet und in der ophthalmologischen Lasertherapievorrichtung 800 angeordnet, dass sie im Verlauf der Therapie während der Erzeugung einer Struktur 1 zur Charakterisierung einer Fläche bzw. eines Volumens des Auges 310 eingesetzt werden können.

Des Weiteren enthält die ophthalmologische Lasertherapievorrichtung 800 wiederum eine einteilige zentrale Steuereinrichtung 500, die über drahtlose, bevorzugt jedoch drahtgebundene Kommunikationswege 510 mit der Einrichtung zur Erzeugung 100 einer Laserstrahlung, also dem Femtosekunden-Lasersystem, und mit der Einrichtung 400 zur Veränderung der Lage des Fokus 130 verbunden ist, und eingerichtet ist, sowohl das Femtosekunden-Lasersystem als auch alle Scanner 411, 412, 413 der Einrichtung 400 zur Veränderung der Lage des Fokus 130 sowie ggf. die Optik 200 zu steuern.

In zweiten Ausführungsbeispiel der erfindungsgemäßen ophthalmologischen Lasertherapievorrichtung 800 umfasst die zentrale Steuereinrichtung 500 eine räumlich nicht von der Steuereinrichtung 500 getrennte Planungseinrichtung 550, die die Funktionen der Planungseinrichtung 550 des ersten Ausführungsbeispiels umfasst, und zusätzlich eine Verarbeitung von Charakterisierungsdaten, die mit der internen Untersuchungseinheit 600 ermittelt wurden, im manuellen wie auch im automatischen Modus erlaubt. Die Planungseinrichtung 550 erlaubt auch eine komplette Neuerstellung eines Scanmusters 140. Die zentrale Steuereinrichtung 500 dieses zweiten Ausführungsbeispiels umfasst zudem eine integrierte Kontrolleinheit 570 des Nachführsystems 700.

Die integrierte Kontrolleinheit 570 bildet in einer ersten Ausführungsvariante - wie in der Fig. 9 gezeigt - zusammen mit der Videokamera 660 das integrierte Nachführsystem 700 der ophthalmologischen Lasertherapievorrichtung 800, mit dem ein zweidimensionaler Korrekturparameter K(ti) bestimmbar ist. Der wiederum erlaubt eine zweidimensionale Nachregelung, d.h., in diesem Fall eine Nachregelung in der lateralen Ebene.

In einer zweiten Ausführungsvariante dieses zweiten Ausführungsbeispiels (nicht in der Fig. 9 gezeigt) bildet die integrierte Kontrolleinheit 570 zusammen mit der optischen Kohärenztomographie (OCT)-Vorrichtung 650 das integrierte Nachführsystem 700 der ophthalmologischen Lasertherapievorrichtung 800, mit dem in diesem Fall ein dreidimensionaler Korrekturparameter K(ti) bestimmbar ist. Dieser wiederum erlaubt eine dreidimensionale, also komplett räumliche Nachregelung der Therapieführung - und damit auch eine Nachregelung in der z-Richtung.

Dabei wird ein Scanmuster 140, das von der Planungseinrichtung 550 der Steuereinrichtung 500 erzeugt oder modifiziert wurde, an die Kontrolleinheit des Nachführsystems 570 übergeben, dort jeweils mit dem Korrekturparameter K(ti) beaufschlagt und in dieser korrigierten Form von der Steuereinrichtung 500 zur Steuerung der ophthalmologischen Lasertherapievorrichtung 800 verwendet. Es wird also nicht die Kalibrierung aktualisiert wie im ersten Ausführungsbeispiel der Fig. 8 beschrieben: In diesem zweiten Ausführungsbeispiel der Fig. 9 findet eine Echtzeit-Neuberechnung des Scanmusters 140 zur Erzeugung der Struktur 1, also beispielsweise des Kapsulotomie-Schnittmusters, statt.

Auch diese Art der Nachregelung der Therapieführung garantiert eine hohe Zielformtreue, selbst wenn sich die Position des Auges 310 relativ zur ophthalmologischen Lasertherapievorrichtung 800 während des Therapiefortschritts ändert.

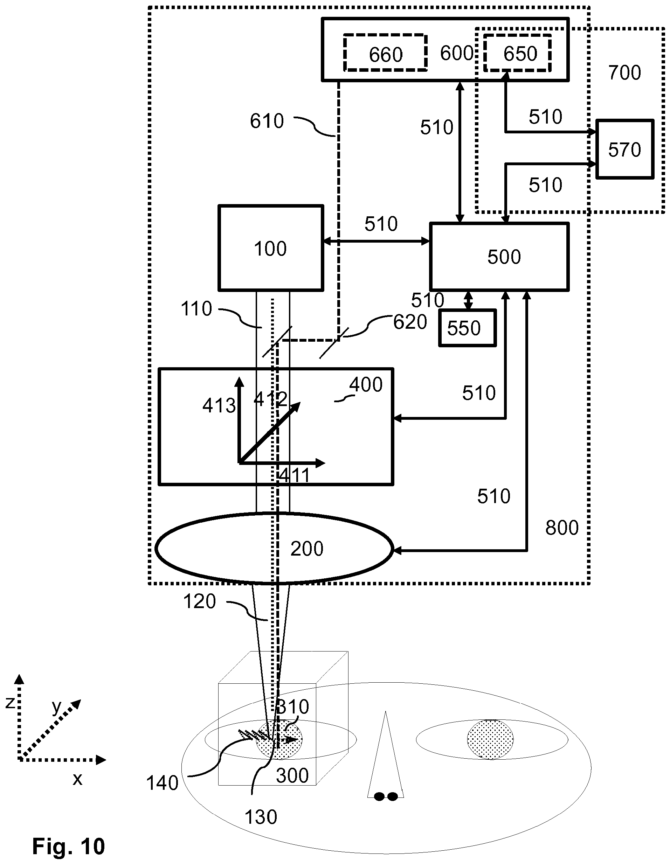

Die Fig. 10 zeigt ein drittes Ausführungsbeispiel einer erfindungsgemäßen ophthalmologischen Lasertherapievorrichtung 800. Viele Erläuterungen, die zu den Ausführungsbeispielen der Fig. 8 und 9 gegeben wurden, treffen auch hier zu. Deshalb soll nur auf die Unterschiede eingegangen werden.

Auch dieses dritte Ausführungsbeispiel enthält eine integrierte Untersuchungseinheit 600 mit zwei Untersuchungseinrichtungen: einer Videokamera 660 und einer optischen Kohärenztomographie (OCT)-Vorrichtung 650. Auch hier sind beide so eingerichtet und in der ophthalmologischen Lasertherapievorrichtung 800 angeordnet, dass sie im Verlauf der Therapie während der Erzeugung einer Struktur 1 zur Charakterisierung einer Fläche bzw. eines Volumens des Auges 310 eingesetzt werden können, und eine Untersuchungsstrahlung 610 auf das Auge 310 gelenkt werden kann, ggf. über Umlenkelemente 620.

Die in der ophthalmologischen Lasertherapievorrichtung 800 integrierte, jedoch von der Steuereinrichtung 500 separierte Planungseinrichtung 550 enthält einen Algorithmus zur Erstellung eines Scanmusters 140 zur Erzeugung einer Struktur 1, mit dem ein solches Scanmuster 140 völlig frei und unabhängig erzeugt werden kann, oder aber ein in einer Auswahltabelle der Planungseinrichtung 550 vorhandenes Scanmuster 140 modifiziert werden kann.

Dieses Scanmuster 140 wird dann an die Steuereinrichtung 500 weitergegeben und von dieser zur Steuerung der Erzeugung der entsprechenden Struktur 1 im Patientenauge 310 mittels Fokusbewegung der Laserstrahlung entlang des Scanmusters 140 genutzt.

Das Nachführsystem 700 dieses dritten Ausführungsbeispiels umfasst eine im Bezug zur ophthalmologischen Lasertherapievorrichtung 800 externe Kontrolleinheit 570, die eingerichtet ist, Bilder und/oder Daten einer internen Untersuchungseinheit 600, 650, 660 zur Charakterisierung einer Fläche oder eines Volumens des Auges 310 zu empfangen und zu verarbeiten, eine Position eines Markers 50 in der Fläche oder in dem Volumen des Auges 310 aus den Bildern und/oder Daten der Untersuchungseinheit 600, 650, 660 zu einem Startzeitpunkt to und zu Folgezeitpunkten tᵢ (i= 1 bis n) der Erzeugung der Struktur 1 zu ermitteln, und aus der Differenz der Position des Markers 50 zum jeweiligen Zeitpunkt tᵢ im Vergleich zum Startzeitpunkt to oder zu einem Zeitpunkt tᵢ₋₁ einen Korrekturparameter K(ti) zu bestimmen.

Die externe Kontrolleinheit 570 bildet nun in einer ersten Ausführungsvariante - wie in der Fig. 10 gezeigt - zusammen mit der internen optischen Kohärenztomographie (OCT)-Vorrichtung 650 das Nachführsystem 700 der ophthalmologischen Lasertherapievorrichtung 800, mit dem in diesem Fall ein dreidimensionaler Korrekturparameter K(ti) bestimmbar ist. Dieser wiederum erlaubt eine dreidimensionale, also komplett räumliche Nachregelung der Therapieführung - und damit auch eine Nachregelung in der z-Richtung.

In einer zweiten Ausführungsvariante dieses dritten Ausführungsbeispiels (nicht in der Fig. 10 gezeigt) bildet die externe Kontrolleinheit 570 zusammen mit der internen Videokamera 660 das Nachführsystem 700 der ophthalmologischen Lasertherapievorrichtung 800, mit dem ein zweidimensionaler Korrekturparameter K(ti) bestimmbar ist. Der wiederum erlaubt eine zweidimensionale Nachregelung, d.h., in diesem Fall eine Nachregelung in der lateralen Ebene.

Dieses dritte Ausführungsbeispiel der ophthalmologischen Lasertherapievorrichtung lässt dem Benutzer zudem die Wahl zwischen einer Nachregelung der Therapieführung über eine Aktualisierung der Kalibrierung durch direkte Änderung der Scannerpositionen oder durch eine Beaufschlagung des Scanmusters 140 mit dem Korrekturparameter K(tᵢ), so dass das Scanmuster 140 in dieser jeweils korrigierten Form von der Steuereinrichtung 500 zur Steuerung der ophthalmologischen Lasertherapievorrichtung 800 verwendet wird bis erneut eine Nachregelung erfolgt.

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar, ohne den Rahmen der vorliegenden Erfindung zu verlassen.

## Patentansprüche

1. Nachführsystem (700) für eine ophthalmologische Lasertherapievorrichtung (800) zur Nachregelung einer Therapieführung zur Erzeugung einer Struktur (1) mittels einer Laserstrahlung (110) der ophthalmologischen Lasertherapievorrichtung (800) in einem Gewebe eines Auges (310), das eine Kontrolleinheit (570) umfasst, wobei die Kontrolleinheit (570) eingerichtet ist, Bilder und/oder Daten einer Untersuchungseinheit (600, 650, 660) zur Charakterisierung einer Fläche oder eines Volumens des Auges (310) zu empfangen und zu verarbeiten, eine Position eines Markers (50), der einen Startpunkt eines Scanmusters (140) umfasst, und/oder aus einem Algorithmus zur Auswertung des bis zum Zeitpunkt tᵢ vollzogenen Scanmusters erzeugt ist, in der Fläche oder in dem Volumen des Auges (310) aus den Bildern und/oder Daten der Untersuchungseinheit (600, 650, 660) zu einem Startzeitpunkt t₀ und zu Folgezeitpunkten tᵢ (i= 1 bis n) der Erzeugung der Struktur (1) zu ermitteln, und aus der Differenz der Position des Markers (50) zum jeweiligen Zeitpunkt tᵢ im Vergleich zum Startzeitpunkt to oder zu einem Zeitpunkt tᵢ₋₁ einen Korrekturparameter K(ti) zu bestimmen, und den Startpunkt des Scanmusters (140) wiederzufinden, um eine geschlossene Struktur (1) zu erzeugen.

2. Nachführsystem (700) nach Anspruch 1, das eingerichtet ist, auf eine interne Untersuchungseinheit (600, 650, 660) der ophthalmologischen Lasertherapievorrichtung (800) zuzugreifen.

3. Nachführsystem (700) nach Anspruch 1 oder 2, das eine Untersuchungseinheit (600, 650, 660) umfasst.

4. Nachführsystem (700) nach einem der Ansprüche 1 bis 3, das eingerichtet ist, mit dem Korrekturparameter K(ti) zum Zeitpunkt tᵢ eine Kalibrierung einer Relativbeziehung des Auges (310) zur ophthalmologischen Lasertherapievorrichtung (800) zu aktualisieren.

5. Nachführsystem (700) nach einem der Ansprüche 1 bis 3, das eingerichtet ist, ein Scanmuster (140), durch das die zeitliche Änderung der Lage eines Fokus (130, 4) der Laserstrahlung (110) im Gewebe des Auges (310) beschrieben ist, zum Zeitpunkt tᵢ mit dem Korrekturparameter K(ti) zu beaufschlagen.

6. Nachführsystem (700) nach Anspruch 5, weiterhin eine Kontrolleinheit (570) umfassend, die für eine Teilkorrektur des Scanmusters (140) eingerichtet ist, durch Beaufschlagung der Lagen des Fokus (130, 4) der Laserstrahlung (110), die in einem Zeitintervall von t > tᵢ bis t ≤ tᵢ₊₁ überstrichen werden, mit dem Korrekturparameter K(tᵢ).

7. Nachführsystem (700) nach einem der Ansprüche 1 bis 6, weiterhin eine Kontrolleinheit (570) umfassend, in die ein Mustererkennungsalgorithmus kodiert ist und/oder das eingerichtet ist zur Erstellung von Differenzbildern.

8. Nachführsystem (700) nach einem der Ansprüche 1 bis 7, wobei der Marker (50) weiterhin eine natürliche Augenstruktur, oder eine künstlich geschaffene Struktur umfasst, und/oder wobei der Marker einteilig oder mehrteilig ausgeführt ist.

9. Nachführsystem (700) nach einem der Ansprüche 1 bis 8, eingerichtet zur Verarbeitung von Bildern und/oder Daten einer Untersuchungseinheit (600, 650, 660), wobei die Untersuchungseinheit (600, 650, 660) mindestens eine der folgenden Untersuchungseinrichtungen umfasst: ein Videosystem, eine optische Kohärenztomographie (OCT)-Vorrichtung, insbesondere ein live-OCT, eine Ultraschallmessvorrichtung, einen konfokalen Detektor und/oder eine Scheimpflug-Kamera.

10. Ophthalmologische Lasertherapievorrichtung (800) umfassend
- eine Einrichtung zur Erzeugung (100) einer Laserstrahlung (110),
- eine Optik (200) zur Fokussierung der Laserstrahlung (110) in einem Fokus (130) in einem Bearbeitungsvolumen (300), insbesondere in einem Gewebe eines Auges,
- eine Einrichtung (400) zur Veränderung der Lage des Fokus (130) im Bearbeitungsvolumen (300),
- eine Steuereinrichtung (500) zur Steuerung der Lasertherapievorrichtung (800), und weiterhin ein Nachführsystem (700) zur Nachregelung einer Therapieführung gemäß einem der Ansprüche 4 bis 9, das eine Untersuchungseinheit (600, 650, 660) umfasst, oder eine interne Untersuchungseinheit (600, 650, 660) zur Charakterisierung des Auges (310) , und ein Nachführsystem (700) gemäß einem der Ansprüche 1 bis 9.

11. Ophthalmologische Lasertherapievorrichtung (800) nach Anspruch 10, umfassend eine Einrichtung zur Erzeugung (100) einer Laserstrahlung (110), die eine gepulste Laserstrahlung, insbesondere eine Femtosekunden-Laserstrahlung, erzeugt.

12. Ophthalmologische Lasertherapievorrichtung (800) nach Anspruch 10 oder 11, umfassend ein Patienteninterface, insbesondere ein Flüssigkeitspatienteninterface oder ein Kontaktglas.

## Claims

1. Tracking system (700) for an ophthalmological laser therapy apparatus (800) for re-adjusting a therapy guidance for generating a structure (1) by means of a laser radiation (110) of the ophthalmological laser therapy apparatus (800) in a tissue of an eye (310), the system comprising a control unit (570), the control unit (570) being configured to receive and process images and/or data from an examination unit (600, 650, 660) for characterizing an area or a volume of the eye (310), to determine a position of a marker (50), which comprises a starting point of a scanning pattern (140), and/or is generated by an algorithm for evaluating the scanning pattern carried out up to the time tᵢ, in the area or in the volume of the eye (310) from the images and/or data of the examination unit (600, 650, 660) at a start time t₀ and at subsequent times tᵢ (i = 1 to n) of the production of the structure (1), and to determine a correction parameter K(tᵢ) from the difference of the position of the marker (50) at the respective time tᵢ in comparison with the start time t₀ or a time tᵢ₋₁, and to retrieve the start point of the scanning pattern (140) in order to generate a closed structure (1).

2. Tracking system (700) according to Claim 1, configured to access an internal examination unit (600, 650, 660) of the ophthalmological laser therapy apparatus (800).

3. Tracking system (700) according to Claim 1 or 2, comprising an examination unit (600, 650, 660).

4. Tracking system (700) according to any one of Claims 1 to 3, configured to update a calibration of a relative relationship of the eye (310) with respect to the ophthalmological laser therapy apparatus (800) by way of the correction parameter K(tᵢ) at the time tᵢ.

5. Tracking system (700) according to any one of Claims 1 to 3, configured to apply, at the time tᵢ, the correction parameter K(tᵢ) to a scanning pattern (140) which describes the change over time in the relative position of a focus (130, 4) of the laser radiation (110) in the tissue of the eye (310).

6. Tracking system (700) according to Claim 5, further comprising a control unit (570) configured to partially correct the scanning pattern (140) by applying the correction parameter K(tᵢ) to the relative positions of the focus (130, 4) of the laser radiation (110) that are swept over during a time interval from t > tᵢ to t ≤ tᵢ₊₁.

7. Tracking system (700) according to any one of Claims 1 to 6, further comprising a control unit (570) in which a pattern recognition algorithm has been encoded and/or been configured to create difference images.

8. Tracking system (700) according to any one of Claims 1 to 7, wherein the marker (50) further comprises a natural eye structure or an artificially created structure and/or wherein the marker has a single-part or multi-part design.

9. Tracking system (700) according to any one of Claims 1 to 8, configured for processing images and/or data from an examination unit (600, 650, 660), the examination unit (600, 650, 660) comprising at least one of the following examination devices: a video system, an optical coherence tomography (OCT) apparatus, in particular a live OCT, an ultrasound measuring apparatus, a confocal detector and/or a Scheimpflug camera.

10. Ophthalmological laser therapy apparatus (800) comprising
- a device for producing (100) a laser radiation (110),
- an optical unit (200) for focusing the laser radiation (110) in a focus (130) in a processing volume (300), more particularly in a tissue of an eye,
- a device (400) for changing the relative position of the focus (130) in the processing volume (300),
- a control device (500) for controlling the laser therapy apparatus (800) and further a tracking system (700) for re-adjusting a therapy guidance according to any one of Claims 4 to 9, which comprises an examination unit (600, 650, 660), or an internal examination unit (600, 650, 660) for characterizing the eye (310), and a tracking system (700) according to any one of Claims 1 to 9.

11. Ophthalmological laser therapy apparatus (800) according to Claim 10, comprising a device for producing (100) a laser radiation (110), which device produces a pulsed laser radiation, in particular a femtosecond laser radiation.

12. Ophthalmological laser therapy apparatus (800) according to Claim 10 or 11, comprising a patient interface, in particular a liquid patient interface or a contact glass.

## Revendications

1. Système suiveur (700) pour un arrangement de thérapie par laser ophtalmologique (800) destiné à réajuster un guidage de thérapie en vue de générer une structure (1) au moyen d'un rayonnement laser (110) de l'arrangement de thérapie par laser ophtalmologique (800) dans un tissu d'un œil (310), lequel comporte une unité de contrôle (570), l'unité de contrôle (570) étant conçue pour recevoir des images et/ou des données d'une unité d'examen (600, 650, 660) en vue de recevoir et de traiter une surface ou un volume de l'œil (310), identifier une position d'un marqueur (50), qui comporte un point de départ d'un modèle de balayage (140), et/ou qui est généré à partir d'un algorithme destiné à interpréter le modèle de balayage accompli jusqu'à l'instant tᵢ, dans la surface ou le volume de l'œil (310) à partir des images et/ou des données de l'unité d'examen (600, 650, 660) à un instant de départ t₀ et aux instants suivants tᵢ (i = 1 à n) de la génération de la structure (1), et à partir de la différence de la position du marqueur (50) à l'instant tᵢ respectif par rapport à l'instant de départ t₀ ou à un instant tᵢ₋₁, déterminer un paramètre de correction K(tᵢ), puis retrouver le point de départ du modèle de balayage (140) afin de générer une structure fermée (1).

2. Système suiveur (700) selon la revendication 1, lequel est conçu pour avoir accès à une unité d'examen (600, 650, 660) interne de l'arrangement de thérapie par laser ophtalmologique (800).

3. Système suiveur (700) selon la revendication 1 ou 2, lequel comprend une unité d'examen (600, 650, 660).

4. Système suiveur (700) selon l'une des revendications 1 à 3, lequel est conçu pour actualiser un étalonnage d'une relation relative de l'œil (310) avec l'arrangement de thérapie par laser ophtalmologique (800) avec le paramètre de correction K(tᵢ) à l'instant tᵢ.

5. Système suiveur (700) selon l'une des revendications 1 à 3, lequel est conçu pour soumettre un schéma de balayage (140), par lequel est décrite la modification dans le temps d'un foyer (130, 4) du rayonnement laser (110) dans le tissu de l'œil (310), au paramètre de correction K(tᵢ) à l'instant tᵢ.

6. Système suiveur (700) selon la revendication 5, comprenant en outre une unité de correction (570), laquelle est conçue pour une correction partielle du schéma de balayage (140) en soumettant au paramètre de correction K(tᵢ) les positions du foyer (130, 4) du rayonnement laser (110) qui sont balayées dans un intervalle de temps de t > tᵢ à t ≤ tᵢ₊₁.

7. Système suiveur (700) selon l'une des revendications 1 à 6, comprenant en outre une unité de correction (570), dans laquelle est codé un algorithme de reconnaissance de modèle et/ou qui est conçue pour créer des images différentielles.

8. Système suiveur (700) selon l'une des revendications 1 à 7, le marqueur (50) comportant en outre une structure d'œil naturelle ou une structure créée artificiellement, et/ou le marqueur étant configuré d'une seule partie ou de plusieurs parties.

9. Système suiveur (700) selon l'une des revendications 1 à 8, conçu pour le traitement d'images et/ou de données d'une unité d'examen (600, 650, 660), l'unité d'examen (600, 650, 660) comprenant au moins l'un des dispositifs d'examen suivants : un système vidéo, un arrangement de tomographie en cohérence optique (OCT), notamment d'OCT en direct, un arrangement de mesure par ultrasons, un détecteur confocal et/ou une caméra Scheimpflug.

10. Arrangement de thérapie par laser ophtalmologique (800), comprenant
- un dispositif destiné à générer (100) un rayonnement laser (110),
- une optique (200) destinée à focaliser le rayonnement laser (110) dans un foyer (130) dans un volume de traitement (300), notamment un tissu d'un œil,
- un dispositif (400) destiné à modifier la position du foyer (130) dans le volume de traitement (300),
- un dispositif de commande (500) destiné à commander l'arrangement de thérapie par laser (800),
et en outre un système suiveur (700) destiné à réajuster un guidage de thérapie selon l'une des revendications 4 à 9, lequel comporte une unité d'examen (600, 650, 660), ou une unité d'examen (600, 650, 660) interne destinée à caractériser l'œil (310) et un système suiveur (700) selon l'une des revendications 1 à 9.

11. Arrangement de thérapie par laser ophtalmologique (800) selon la revendication 10, comprenant un dispositif destiné à générer (100) un rayonnement laser (110), lequel génère un rayonnement laser pulsé, notamment un rayonnement laser femtosecondes.

12. Arrangement de thérapie par laser ophtalmologique (800) selon la revendication 10 ou 11, comprenant une interface de patient, notamment une interface de patient liquide ou un verre de contact.
